(19) Europäisches Patentamt
European Patent Office
Office européen des brevets

(11) EP 3 766 959 A1

(12) **EUROPEAN PATENT APPLICATION**
published in accordance with Art. 153(4) EPC

(43) Date of publication:
**20.01.2021 Bulletin 2021/03**

(21) Application number: **19767089.6**

(22) Date of filing: **15.03.2019**

(51) Int Cl.:
***C12N 1/04*** *(2006.01)* ***C12M 1/26*** *(2006.01)*
***C12Q 1/02*** *(2006.01)*

(86) International application number:
**PCT/JP2019/011001**

(87) International publication number:
**WO 2019/177166 (19.09.2019 Gazette 2019/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2018 JP 2018048606**

(71) Applicant: **Metabologenomics, Inc.**
**Tsuruoka-shi, Yamagata 997-0052 (JP)**

(72) Inventors:
- **MURAKAMI, Shinnosuke**
**Tsuruoka-shi, Yamagata 997-0052 (JP)**
- **ITO, Masaki**
**Tsuruoka-shi, Yamagata 997-0052 (JP)**
- **MORI, Yuka**
**Tsuruoka-shi, Yamagata 997-0052 (JP)**
- **NISHIMOTO, Yuichiro**
**Tokyo 152-8550 (JP)**
- **FUKUDA, Shinji**
**Tsuruoka-shi, Yamagata 997-0052 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Straße 22**
**85354 Freising (DE)**

(54) **FECAL STORAGE METHOD**

(57) The present invention provides a non-cryopreservation method that enables inexpensive and stable storage of feces. More specifically, the present invention provides: (A) a storage method of feces, the storage method including drying the feces in the presence of a solid desiccant; (B) a hermetic container including: (a) a solid desiccant; and (b) feces under the non-contact state with the solid desiccant; and (C) a feces fixing article including: (a) one or more members containing or fixing a solid desiccant; and (b) feces under the contact state with the solid desiccant. As the solid desiccant, a water absorbing solid desiccant such as silica gel is preferred.

*FIG. 1*

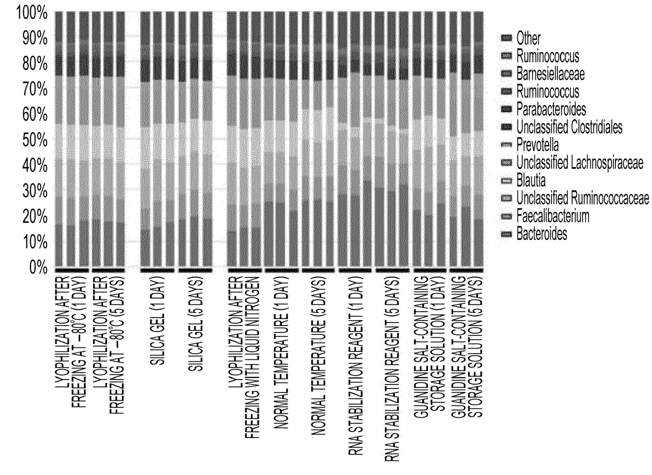


EP 3 766 959 A1

## Description

### Technical Field

The present invention relates to a storage method of feces.

### Background Art

Many intestinal microbes exist in human intestinal tracts and form complicated intestinal microbiota. In recent years, it has been reported that intestinal microbiota can be involved in various human physiological functions and diseases. Accordingly, evaluation of the intestinal environment(s) has been expected to be made possible by subjecting feces to both microbiome analysis that qualitatively or quantitatively evaluates intestinal microbes (for example, metagenomic analysis, and metatranscriptomic analysis), and metabolomic analysis that qualitatively or quantitatively evaluates intestinal metabolite(s) to analyze the obtained information in an integrated manner.

Incidentally, to stably store and appropriately analyze intestinal microbes and intestinal metabolite(s) contained in feces, it is desirable to freeze feces immediately after collection and maintain a temperature condition of less than 0°C (Non-Patent Literature 1). However, storage and transportation of fecal samples by cryopreservation require enormous costs due to preparation of equipment capable of freezing (particularly, in a case of storage and transportation for a long period of time) and electric power consumption for freezing. Further, there is a possibility that precious samples are damaged due to unexpected dissolution treatment of cryopreserved feces, and as a result, evaluation by microbiome analysis and/or metabolomic analysis with high accuracy becomes difficult. Thus, development of a non-cryopreservation method that enables more inexpensive and stable storage of fecal samples has been desired.

A guanidine thiocyanate-containing solution has been hitherto reported as a solution that can stably store DNA in feces at normal temperature (Non-Patent Literature 2).

### Citation List

### Non-Patent Literature

Non-Patent Literature 1: Murakami et al., Evidence-Based Complementary and Alternative Medicine, 2015, pp. 824395.

Non-Patent Literature 2: Nishimoto et al., Gut., 2016, 65(9): 1574-5

### Summary of Invention

### Technical Problem

An object of the present invention is to develop a non-cryopreservation method that enables inexpensive and stable storage of feces.

### Solution to Problem

The present inventors conducted diligent studies and conceived that the presence of water is indispensable for metabolic reaction and decomposition of substance by general organisms (for example, hydrolysis), and therefore removal of water in feces is effective for maintaining the composition of metabolite(s) immediately after defecation. Thus, the present inventors studied various drying methods to remove water in feces, and found that drying using a solid desiccant enables more stable storage of genes in feces than the conventional art (guanidine thiocyanate-containing solution) (Figs.1 to 3).

Moreover, there has been a problem that, when fecal samples are stored by the conventional art (guanidine thiocyanate-containing solution), analysis of ionic metabolite(s) by capillary electrophoresis (CE)-time-of-flight mass spectrometry (TOFMS) is inapplicable due to the salt concentration. According to the present invention, adjustment of the salt concentration is not necessary, and it is therefore conceived that the above-described problem with the salt concentration can be solved.

The present inventors completed the present invention based on the above-described finding. That is, the present invention is as follows.

[1] A storage method of feces, the storage method including drying the feces in presence of a solid desiccant.
[2] The storage method according to [1], in which the feces are feces of animals, fishes or insects.
[3] The storage method according to [1] or [2], in which the solid desiccant is a water absorbing solid desiccant.
[4] The storage method according to [3], in which the water absorbing solid desiccant is silica gel.
[5] The storage method according to any of [1] to [4], in which the drying of the feces is performed under a contact state with the solid desiccant, and
a contact area between the feces and the solid desiccant is 100 ($mm^2$/1 g of feces) or more.
[6] The storage method according to any of [1] to [4], in which the drying of the feces is performed under a non-contact state with the solid desiccant, and
a weight ratio of the feces to the solid desiccant (solid desiccant/feces) is 0.1 to 10,000.
[7] The storage method according to any of [1] to [6], in which the drying is performed at a temperature higher than 0°C.
[8] The storage method according to [7], in which the drying is performed at 4 to 60°C.
[9] The storage method according to [7], in which the drying is performed by heating using a heating device.
[10] The storage method according to [9], in which the heating device is a microwave irradiator.
[11] An analysis method of feces, the method including:

(1) drying the feces in presence of a solid desiccant; and
(2) analyzing the dried feces.

[12] The method according to [11], in which analysis is performed on an intestinal bacteria and/or substance in the feces.
[13] The method according to [12], in which the intestinal bacteria is one or more types of microorganisms selected from the group consisting of microorganisms belonging to Clostridiales and microorganisms belonging to Parabacteroides.
[14] The method according to [12], in which the intestinal bacteria is one or more types of microorganisms selected from the group consisting of Firmicutes, Bacteroidetes, Proteobacteria, Actinobacteria, and Verrucomicrobia.
[15] The method according to [12], in which the intestinal bacteria is one or more types of microorganisms selected from the group consisting of Bacteroides, Blautia, Prevotella, Ruminococcus, and Barnesiellaceae.
[16] The method according to any one of [12] to [15], in which the substance includes one or more types of substances selected from the group consisting of:

(1) 2-hydroxyglutarate;
(2) 2-hydroxypentanoate;
(3) 2-aminobenzamide;
(4) 3-methylbutanoate;
(5) 4-(β-acetylaminoethyl)imidazole;
(6) 4-methyl-5-thiazole ethanol;
(7) 5-aminopentanoate;
(8) 7-methylguanine;
(9) adipate;
(10) alanyl alanine;
(11) α-aminoadipate;
(12) anserine;
(13) aspartate;
(14) azelate;
(15) butyrate;
(16) carnitine;
(17) cholate;
(18) choline;
(19) citramalate;
(20) citrate;
(21) citrulline;
(22) deoxycholate;
(23) diethanolamine;
(24) dodecanedioate;
(25) γ-butyrobetaine;
(26) glutamate;
(27) glutarate;

(28) glycyl leucine;
(29) glycerophosphate;
(30) glycolate;
(31) guanine;
(32) hexanoate;
(33) histamine;
(34) homoserine;
(35) hypoxanthine;
(36) imiadzole-4-acetate;
(37) isopropanolamine;
(38) lactate;
(39) lysine;
(40) malate;
(41) malonate;
(42) methionine;
(43) N-acetylaspartate;
(44) N-acetylglutamate;
(45) N-methylalanine;
(46) N-methylglutamate;
(47) N1-acetylspermidine;
(48) N1, N8-diacetylspermidine
(49) ornithine
(50) pelargonate
(51) pentanoate;
(52) phthalate;
(53) pimelate;
(54) pipecolate;
(55) proline;
(56) proline betaine;
(57) propionate;
(58) pyridoxamine;
(59) sebacate;
(60) serine;
(61) succinate;
(62) terephthalate;
(63) thiamine;
(64) trigonelline;
(65) trimethylamine; and
(66) tryptophan.

[17] A hermetic container including: (a) a solid desiccant; and (b) feces under a non-contact state with the solid desiccant.
[18] The hermetic container according to [17], in which a weight ratio of the feces to the solid desiccant (solid desiccant/feces) is 0.1 to 10,000.
[19] The hermetic container according to [17] or [18], in which the solid desiccant is a water absorbing solid desiccant.
[20] The hermetic container according to [19], in which the water absorbing solid desiccant is silica gel.
[21] The hermetic container according to any of [17] to [20], in which the feces are dried.
[22] A feces fixing article including: (a) one or more members containing or fixing a solid desiccant; and (b) feces under a contact state with the solid desiccant.
[23] The feces fixing article according to [22], including: (a1) a first plate having a first holding surface; (a2) a second plate having a second holding surface; and (b) the feces, in which

the feces are sandwiched between the first and second holding surfaces, which are disposed to face each other, to be in contact with the solid desiccant, and
a contact area between the feces and a holding surface containing the solid desiccant is 100 ($mm^2$/1 g of feces) or more.

[24] The feces fixing article according to [22] or [23], further including (c) a spacer between the first and second holding surfaces disposed facing each other.

[25] The feces fixing article according to any of [22] to [24], in which at least one of a first member and a second member is a water absorbing solid desiccant plate.

[26] The feces fixing article according to [25], in which the water absorbing solid desiccant plate is a silica gel plate.

[27] The feces fixing article according to any of [22] to [26], in which the feces are dried.

## Advantageous Effects of Invention

According to the present invention, feces can be stored inexpensively and stably. Further, according to the present invention, feces can be rapidly dried. Further, according to the present invention, exposure of feces to the outside can be minimized. This allows contact of feces with the outer environment to be prevented and provides ease in handling, and thus it is suitable for storage and transportation of feces.

Also, the feces stored by the present invention can be suitably used for both microbiome analysis and metabolomic analysis.

## Brief Description of Drawings

Fig. 1 illustrates 100% stacked bar graphs for analysis results in the genus level of analysis results of intestinal microbiota in each sampling condition. "Lyophilization after freezing at -80°C" (conventional condition: lyophilization described in Non-Patent Literature 1) is shown on the leftmost side, and then the analysis results of intestinal microbiota obtained by the non-cryopreservation method of the present invention using silica gel are shown.

Fig. 2 illustrates 100% stacked bar graphs for analysis results in the division level of analysis results of intestinal microbiota in each sampling condition.

Fig. 3 is a graph showing the average value and standard deviation (SD) of weighted UniFrac distance between samples with various storage condition. The same fecal samples were placed under various storage conditions, and then analysis of microbiota was conducted through DNA extraction. Analysis was conducted by using three samples (technical replicate) for each storage condition. The graph shows the average value and standard deviation (SD) of weighted UniFrac distance for three samples of "lyophilization after freezing at -80°C (1 day)" and all combinations of three samples for each condition (n = 9). Only the bar graph of "lyophilization after freezing at - 80°C (1 day)" indicates the average value and standard deviation of n = 6 excluding a distance between the same samples.

Fig. 4 is a graph showing comparison of metabolite(s) extracted from the same fecal samples under different drying conditions. The concentration of respective metabolite(s) calculated for "lyophilization after freezing at -80°C (5 days)" (conventional condition) and "silica gel (5 days)" (condition of the present invention) were represented by a scatter plot.

Fig. 5 is a graph showing a comparison (25°C) of the water contents of unfrozen feces and freeze-thawed feces relative to the drying time. The "water content (before treatment)" was determined from a weight difference between the sample feces before and after lyophilization.

Fig. 6 is a graph showing transition (4°C) of the water content of sample feces relative to the drying time. The "water content (before treatment)" was determined from a weight difference between the sample feces before and after lyophilization.

Fig. 7 is a graph showing change over time of the temperature of sample feces when a disposable body warmer is used.

Fig. 8 is a graph showing change over time of the water content of sample feces sandwiched between plate-like silica gel at a high temperature condition (50°C or higher). The "water content (before treatment)" was determined from a weight difference between the sample feces before and after lyophilization.

Fig. 9 is a graph showing change over time of the water content of sample feces sandwiched between plate-like silica gel in a case of heat-treating in a microwave oven. The "water content (before treatment)" was determined from a weight difference between the sample feces before and after lyophilization.

Fig. 10 is a graph showing change over time of the water content of sample feces sandwiched between plate-like silica gel at a time of enclosing a deoxidizer or not enclosing a deoxidizer. The "water content (before treatment)" was determined from a weight difference between the sample feces before and after lyophilization.

## Description of Embodiments

The present invention provides a storage method of feces, including drying feces in the presence of a solid desiccant. The storage method of the present invention allows feces to be dried and stored without freezing, and thus is different in the idea from the conventional storage method which lyophilizes feces.

As feces, feces derived from any organisms can be used. Examples of such organisms include animals, fishes, and insects, and animals are preferred. Examples of the animal include mammals, birds, reptilians, and amphibians, and mammals are preferred. Examples of the mammal includes primates (for example, humans, monkeys, and chimpanzees), rodents (for example, mice, rats, hamsters, guinea pigs, and rabbits), and livestock and working mammals (for example, cattle, pigs, sheep, goats, and horses). Preferably, feces are human feces.

Feces can be used in any shape and state. For example, in a case where feces are dried under the contact state with a solid desiccant, feces can be crushed into a flat plate shape to improve a contact area with the solid desiccant. Also, feces can be brought into contact with the solid desiccant in a state of being one mass or divided into a plurality of masses. In a case where feces are dried under the non-contact state with a solid desiccant, the feces may be crushed to improve the proportion of surface area relative to the weight of the feces, or may also be divided into a plurality of masses.

The amount of feces used in the present invention is not particularly limited, and may be appropriately set according to whether feces are dried under the contact state with a solid desiccant or under the non-contact state with a solid desiccant. In a case where feces are dried under the contact state with a solid desiccant, the amount of feces is, for example, 0.01 to 10.0 g, preferably 0.02 to 5.0 g, more preferably 0.03 to 1.0 g, even more preferably 0.04 to 0.5 g, and particularly preferably 0.05 to 0.3 g. In a case where feces are dried under the non-contact state with a solid desiccant, the amount of feces is preferably small. In such a case, the amount of feces is, for example, 0.01 to 1.0 g, preferably 0.01 to 0.8 g, more preferably 0.01 to 0.6 g, even more preferably 0.01 to 0.4 g, and particularly preferably 0.01 to 0.3 g.

As the solid desiccant, for example, any solid desiccant can be used. Examples of such a solid desiccant include water absorbing solid desiccants, and sheet-like desiccants, and the water absorbing solid desiccants are preferred. Examples of the water absorbing solid desiccant include silica gel, alumina silica gel, allophane, molecular sieve, zeolite, lime, calcium chloride, diatomaceous earth, and water absorbing polymers. Among water absorbing solid desiccants, silica gel, alumina silica gel, and allophane are preferred, and silica gel is more preferred.

As the solid desiccant, those having any shape can be used. Examples of such a shape include a plate, particles (for example, solid substances such as spheres having a diameter of 2 to 5 mm), and powder. From the viewpoint of ease in operation or the like, the plate may also be used as the solid desiccant.

In an embodiment, drying of feces can be performed under the contact state with a solid desiccant. In such a case, contact of the feces with solid desiccant can be performed so as to sufficiently promote drying of the feces. For example, contact can be performed by appropriately setting conditions such as the contact manner, contact area, temperature, and contact time.

The contact of feces and solid desiccant can be performed by any contact manner. For example, in a case where a plate is used as a solid desiccant, contact can be performed by placing feces on a plate or sandwiching feces between two or more plates. In a case where particles or powder are used as a solid desiccant, contact can be performed by covering feces with many particles or a large amount of powder (for example, embedding feces in many particles or a large amount of powder). Also, in a case where one or more members containing a solid desiccant, which is described later, in a feces fixing article of the present invention are used, feces can be treated in a later-described manner.

Contact of the feces and solid desiccant can be performed so as to secure a sufficient contact area from the viewpoint of sufficiently promoting drying of feces. Such a contact area may vary depending on factors such as the types and amounts of feces and solid desiccant, temperature, and contact period, but may also be, for example, 100 ($mm^2$/1 g of feces) or more. The contact area may also be preferably 500 ($mm^2$/1 g of feces) or more, more preferably 1,000 ($mm^2$/1 g of feces) or more, even more preferably 1,600 ($mm^2$/1 g of feces) or more, and particularly preferably 2,000 ($mm^2$/1 g of feces) or 2,500 ($mm^2$/1 g feces) or more. The contact area may also be 10,000 ($mm^2$/1 g of feces) or less, 8,000 ($mm^2$/1 g of feces) or less, 6,000 ($mm^2$/1 g of feces) or less, or 4,000 ($mm^2$/1 g of feces) or less. More specifically, such a contact area may also be 500 to 10,000 ($mm^2$/1 g of feces), 800 to 8,000 ($mm^2$/1 g of feces), more preferably 1,200 to 6,000 ($mm^2$/1 g of feces), even more preferably 1,600 to 4,000 ($mm^2$/1 g of feces), and particularly preferably 2,000 to 4,000 ($mm^2$/1 g of feces) or 2,500 to 4,000 ($mm^2$/1 g of feces).

The temperature for drying can be set to a temperature at which water does not freeze (i.e., temperature higher than 0°C). To achieve drying more simply, drying can be performed under a non-heating condition (for example, air temperature, room temperature, or normal temperature). According to the storage method of the present invention, it is verified that feces can be sufficiently dried in a temperature range of 4°C or higher. Accordingly, in the present invention, feces can be dried in such a temperature range (for example, 4 to 60°C, 10 to 45°C, 15°C to 37°C, or 20 to 35°C). The temperature for drying may also be a high temperature. For example, drying of feces may be promoted by heating using a heating device. Examples of the heating device include a microwave irradiator (for example, a microwave oven), a hot plate, and an oven. Preferably, as a drying device, a microwave irradiator (for example, a microwave oven) can be used which can instantaneously kill microorganisms and deactivate enzymes in feces and dry feces by irradiation with microwaves.

In another embodiment, drying of feces can be performed under the non-contact state with a solid desiccant.

In such a case, drying may also be performed in a closed system. Examples of the closed system include sealable containers (for example, a tube, bottle, and case). In such a case, the weight ratio of feces to solid desiccant (solid desiccant/feces) is not particularly limited as long as the feces are sufficiently dried under the non-contact state with the solid desiccant, but may be, for example, 0.1 to 10,000, preferably 0.5 to 5,000, more preferably 1 to 1,000, even more preferably 5 to 500, and particularly preferably 10 to 300.

Drying of feces may also be performed through sealing utilizing outer packages (for example, a bag, pouch, and sheet) even in either a contact state or non-contact state. For example, a sampling kit after sampling is set in a container supporting a solid desiccant on a wall surface thereof so as not to be in contact, and this may be sealed with an outer package such as a plastic bag with high gas barrier properties (for example, Ziploc (registered trademark)). Alternatively, a sampling kit after sampling may also be protected and dried in a non-sealing-type protection case supporting a solid desiccant (for example, a fitting-type case or a cap-type case such as a protection case for the tip end of a toothbrush). As the sampling kit, any tool that can sample feces may be used. For example, a rod having a sampling part at the tip end of the handle (as a shape, one having one side serving as a function part like a cotton swab or a toothbrush) may also be utilized. As the shape of the handle of the sampling kit, those having various shapes such as a rod-like shape, a spatula-like shape, a scissor-like shape, a glove, and a mitten can be utilized.

Drying time significantly varies depending on factors such as the types and amounts of feces and solid desiccant, and temperature. According to the present invention, the drying time may also be, for example, 5 minutes or more, preferably 10 minutes or more, more preferably 20 minutes or more, even more preferably 30 minutes or more, and particularly preferably 40 or 50 minutes or more.

The degree of drying of feces is not particularly limited as long as the water content significantly decreases compared to feces before drying, and differs depending on factors such as the original water content of feces used, and a desired storage period for the feces. When description is made by exemplifying human feces (solid feces) as the feces, the water content of human feces is normally approximately 70 to 85% by weight. Accordingly, "drying" of feces can be determined with decrease of water content of feces to 30% by weight or less being as a reference. However, since further decrease of the water content is desired for stable storage of feces for a long period of time, the water content of dried feces is preferably 20% by weight or less, more preferably 10% by weight or less, even more preferably 8% by weight or less, and particularly preferably 5% by weight or less. Measurement of the water content of feces can be determined on the basis of the mass of water lost by lyophilizing feces and determining the mass of water lost from a difference in weight change before and after lyophilization.

According to the storage method of the present invention, feces can be stored in a dry state while contact or non-contact of the feces and solid desiccant is maintained after drying feces. Thus, a long storage period can be set after drying feces. As such a storage period, for example, 1 day or more, 10 days or more, 20 days or more, 1 month or more, 3 months or more, 6 months or more, 1 year or more, or 3 years or more can be presumed.

The present invention also provides an analysis method of feces including the following:

(1) drying the feces in presence of a solid desiccant; and
(2) analyzing the dried feces.

The above-described step (1) can be performed in the same manner as the storage method of the present invention.

In the above-described step (2), analysis of the feces obtained in the above-described step (1) can be performed. Feces can be treated before the analysis of feces. Examples of such a treatment method of feces include a method of subjecting feces to crushing and suspending treatment with beads to obtain a suspension. As such a treatment method, any publicly known method in this field can be employed (for example, WO 2017/043087 A; Murakami, S. et al., "The Consumption of Bicarbonate-Rich Mineral Water Improves Glycemic Control." Evidence-Based Complementary and Alternative Medicine; 2015, pp.824395).

For example, analysis can also be performed on an intestinal bacteria in feces. Such analysis of intestinal bacteria is known as, for example, metagenomic analysis, and metatranscriptomic analysis. Thus, analysis of intestinal bacteria in feces can be performed by, for example, a method such as a metagenomic analysis method publicly known in the field (for example, Non-Patent Literature 1; WO 2017/043087 A). Such analysis can be performed through, for example, nucleic acid (DNA and/ or RNA) extraction and on the basis of the sequence of extracted nucleic acid.

In the analysis method of the present invention, an intestinal bacteria in optional feces in feces can be analyzed, but one or more types of intestinal microbes as shown in Examples described later can also be analyzed. Among the intestinal microbes, microorganisms belonging to Clostridiales and Parabacteroides are identified as intestinal microbes whose ratios cannot vary depending on the drying method. Thus, from the viewpoint of simple and general comparison and study of data of intestinal bacteria regardless of the drying and storage method, it is preferred to analyze, as the intestinal bacteria in feces, one or more types of microorganisms selected from the group consisting of microorganisms belonging to Clostridiales and microorganisms belonging to Parabacteroides. Examples of the microorganism belonging to Clostridiales include microorganisms belonging to Ruminococcaceae or Lachnospiraceae. Examples of the microorganism belonging to Ruminococcaceae include Ruminococcus or Faecalibacterium, Acetanaerobacterium, Hydrogenoanaerobacterium, Oscillospira, Papillibacter, Ruminoclostridium, and Subdoligranulum. Examples of the microorganism belonging to Lachnospiraceae include Acetitomaculum, Anaerostipes, Butyrivibrio, Coprococcus, Dorea, Eisenbergiella, Fusicatenibacter, Lachnoclostridium, Lachnospira, Marvinbryantia, Moryella, Pseudobutyrivibrio, Robinsoniella, Roseburia, Sellimonas, Shuttleworthia, and Tyzzerella.

Further, in the analysis method of the present invention, one or more types of intestinal microbes may also be analyzed whose existing ratios change in non-cryopreservation methods other than the storage method of the present invention (for example, storage at normal temperature or storage using an RNA stabilization reagent) compared to the conventional cryopreservation method (lyophilization as described in Non-Patent Literature 1), but can be secured in the storage method of the present invention at a level equivalent to that of the conventional cryopreservation method. Examples of such intestinal microbes include one or more types of microorganisms selected from the group consisting of Firmicutes, Bacteroidetes, Proteobacteria, Actinobacteria, and Verrucomicrobia. More specific examples include microorganisms belonging to Bacteroidaceae, Lachnospiraceae, Prevotellaceae, Ruminococcaceae, and Barnesiellaceae. Examples of the microorganism belonging to Bacteroidaceae include microorganisms belonging to Bacteroides. Examples of the microorganism belonging to Lachnospiraceae include microorganisms belonging to Blautia. Examples of the microorganism belonging to Prevotellaceae include microorganisms belonging to Prevotella. Examples of the microorganism belonging to Ruminococcaceae include microorganisms belonging to Ruminococcus.

Of course, for intestinal microbes other than the microorganisms specifically described above, comparison and study of data are possible as well by considering factors such as the variation rate by the drying method. The number of microorganisms to be analyzed in the present invention is not particularly limited, and may be, for example, one or more types, two or more types, five or more types, ten or more types, or 20 or more types. Analysis of the microorganism can be performed in any level of order, family, genus, or species.

Analysis may also be performed on a substance in feces. Analysis of such a substance is known as metabolomic analysis. Accordingly, analysis of a substance in feces can be performed by, for example, a method such as a metabolomic analysis method publicly known in the field (for example, Non-Patent Literature 1; Mishima et al., Kidney Int. 2017 Sep; 92(3): 634 to 645; WO 2017/043087 A). For example, such analysis can be performed by appropriately combining methods such as chromatography (for example, liquid chromatography, and gas chromatography), capillary electrophoresis, mass spectrometry, nuclear magnetic resonance spectroscopy, and matrix-assisted laser desorption-ionization mass spectrometry.

An optional substance in feces can be analyzed in the analysis method of the present invention, and one or more types of substances selected from the group consisting of substances shown in Examples described later may also be analyzed. Preferably, substances as described below, of which the variation of amount is small regardless of the drying and storage method, are analyzed.

(1) 2-hydroxyglutarate
(2) 2-hydroxypentanoate
(3) 2-aminobenzamide: 2AB
(4) 3-methylbutanoate
(5) 4-(β-acetylaminoethyl)imidazole
(6) (4-methyl-5-thiazoleetanol)
(7) (5-aminovalerate)
(8) 7-methylguanine
(9) adipate
(10) alanyl alanine (Ala-Ala)
(11) α-aminoadipate
(12) anserine
(13) aspartate (Asp)
(14) azelate
(15) butyrate
(16) carnitine
(17) cholate
(18) choline
(19) citramalate
(20) citrate
(21) citrulline
(22) deoxycholate (DCA)
(23) diethanolamine

(24) dodecanedioate
(25) γ-butyrobetaine
(26) glutamate (Glu)
(27) glutarate
(28) glycyl leucine (Gly-Leu)
(29) glycerophosphate
(30) glycolate
(31) guanine
(32) hexanoate
(33) histamine
(34) homoserine
(35) hypoxanthine
(36) imiadzole-4-acetate
(37) isopropanolamine
(38) lactate
(39) lysine (Lys)
(40) malate
(41) malonate
(42) methionine (Met)
(43) N-acetylaspartate
(44) N-acetylglutamate
(45) N-methylalanine
(46) N-methylglutamate
(47) N1-acetylspermidine
(48) N1, N8-diacetylspermidine
(49) ornithine
(50) pelargonate
(51) pentanoate
(52) phthalate
(53) pimelate
(54) pipecolate
(55) proline (Pro)
(56) proline betaine
(57) propionate
(58) pyridoxamine
(59) sebacate
(60) serine (Ser)
(61) succinate
(62) terephthalate
(63) thiamine
(64) trigonelline
(65) trimethylamine
(66) tryptophan (Trp)

These substances are identified as metabolite(s) of which the change in amount is small regardless of the drying method. Thus, from the viewpoint of comparison and study using simple, generic, and highly reliable data of metabolite(s) regardless of the drying and storage method, one or more types of substances as described above are preferably analyzed as a substance in feces. As a substance to be analyzed among the above-described substances, a substance having a variation ratio of 0.3 or more is preferred, a substance having a variation ratio of 0.4 or more is more preferred, a substance having a variation ratio of 0.5 or more is even more preferred, and a substance having a variation ratio of 0.6 or more, 0.7 or more, or 0.8 or more is particularly preferred (see, Examples). As a substance to be analyzed among the above-described substances, a substance having a variation ratio of 3.0 or less is preferred, a substance having a variation ratio of 2.5 or less is more preferred, a substance having a variation ratio of 2.0 or less is even more preferred, and a substance having a variation ratio of 1.5 or less, 1.35 or less, or 1.2 or less is particularly preferred (see, Examples). More specifically, as a substance to be analyzed among the above-described substances, a substance having a variation ratio of 0.3 or more and 3.0 or less is preferred, a substance having a variation ratio of 0.4 or more and 2.5 or less is more preferred, a substance having a variation ratio of 0.5 or more and 2.0 or less is even more preferred, and a substance having a variation ratio of 0.6 or more and 1.5 or less, 0.7 or more and 1.35 or less, or 0.8 or more and 1.2 or less is particularly preferred (see, Examples). Of course, comparison and study of data for substances other than these substances are possible as well by considering factors such as the variation ratio by the drying method. Alternatively, specific substances, in which the relationship between health and the intestinal bacteria are known or indicated, may also be analyzed. Examples of such a specific substance include short-chain fatty acids having 6 or less of carbon atoms (for example, propionate, and butyrate), bile acids (for example, cholate, and deoxycholate), and trimethylamine. The number of substances to be analyzed in the present invention is not particularly limited, and may be, for example, one or more types, two or more types, five or more types, ten or more types, 20 or more types, 50 or more types, or 100 or more types.

The present invention provides an article that can be suitably used for the storage method of the present invention.

In an embodiment, an article that can be suitably used for the storage method of the present invention is a hermetic container including: (a) a solid desiccant; and (b) feces under the non-contact state with the solid desiccant. Such a sealing container is useful for suitably drying and storing feces under the non-contact state with a solid desiccant. The solid desiccant, feces, and hermetic container are as described above.

The weight ratio of feces to solid desiccant in the sealing container (solid desiccant/feces) is not particularly limited as long as the feces are sufficiently dried under the non-contact state with the solid desiccant. The weight ratio may also be, for example, 0.1 to 10,000, preferably 0.5 to 5,000, more preferably 1 to 1,000, even more preferably 5 to 500, and particularly preferably 10 to 300.

Another embodiment provides, as an article preferably used for the storage method of the present invention, a feces fixing article including: (a) one or more members containing or fixing a solid desiccant; and (b) feces in which the feces are in contact with the solid desiccant. Such a feces fixing article is useful for suitably drying and storing feces under the contact state with the solid desiccant. The solid desiccant, feces, and hermetic container are as described above.

The member containing a solid desiccant may be a formed member of a solid desiccant, which essentially consists of only a solid desiccant, or may be a member composed of a mixture of a solid desiccant and another material. Examples and preferred examples of the solid desiccant are as described above. As another material, an optional material capable of holding the solid desiccant can be used. Examples of such another material include metal, plastic, glass, a film, and a filter. From the viewpoint of utilizing a simple member or the like, the member containing a solid desiccant may also be a formed member of a solid desiccant, which essentially consists of only a solid desiccant.

As the member, an optional member capable of fixing or accommodating feces, or adsorbing feces can be used. Examples of such a member include articles capable of fixing feces (for example, a plate), articles capable of adsorbing feces (for example, particles), and articles capable of accommodating stool(s) (for example, a container having a rectangular columnar, columnar, or triangular prism shape (for example, a tube, bottle, and case), and the sampling kit described above). The number of the members is not particularly limited as long as the number is one or more, and the number varies according to the type of the member. For example, in a case where the member is a plate, one plate or two plates can be used, for example. On the other hand, in a case where the member is a particle, a sufficient number of particles for covering feces can be used. Also, in a case where the member is a container, feces may be accommodated in one container, or divided and individually accommodated in a plurality of containers. In a case where two or more members are used, respective members may be the same or different. For example, in a case where two plates are used, a recess may be provided in a predetermined portion of one plate so as to facilitate accommodation of feces in the predetermined portion, and no recess may be provided in the other plate so as to serve as a cover. From the viewpoint of a simple operation and ease in handling or the like, a plate may be used as the member. The feces fixing article is also preferably sealed by utilizing an outer package as described above. Alternatively, a feces fixing article (for example, a sampling kit after sampling) is preferably protected and dried in a non-sealing-type protection case as described above.

Preferably, as the member containing a solid desiccant, a member that has a holding surface of feces and contains a solid desiccant in the holding surface can be used. Examples of such a member include a plate, a container, and a sampling kit. The plate is preferred from the viewpoint of, for example, more easily preparing and procuring the member containing a solid desiccant.

Preferably, the feces fixing article of the present invention includes: (a1) a first plate having a first holding surface; (a2) a second plate having a second holding surface; and (b) feces, in which the feces are sandwiched between the first and second holding surfaces, which are disposed to face each other, to be in contact with the solid desiccant, and a contact area between the feces and the holding surface containing the solid desiccant is 100 ($mm^2$/1 g of feces) or more.

Such a feces fixing article can securely hold feces with holding surfaces of two plates. Also, since the feces and solid desiccant are in contact with a sufficient contact area, efficient water absorbing is made possible, and thus feces can be rapidly dried. Further, feces are sandwiched between the first and second plates (first and second holding surfaces), and thus exposure of feces to the outer environment is small. Such a feces fixing article can prevent contact of feces with the outer environment and is easily handled, and thus is suitable for storage and transportation of feces.

The above-described plate may be a formed member of a solid desiccant or a member composed of a mixture of a solid desiccant and another material. The plate may also be a formed member of a solid desiccant from the viewpoint of utilizing a simple member or the like. The plate is preferably a water absorbing solid desiccant plate, and more preferably a silica gel plate. In a case where the first and second plates are used, the plates may be the same or different as described above. The same plates are preferable from the viewpoint of, for example, general use of uniform plates.

The thickness of the plate is not particularly limited as long as the plate can have a thickness that can secure an appropriate hardness from the viewpoint of, for example, exerting a function as a desiccant (for example, water absorbing properties), ease in handling, or the like. The thickness is, for example, 1.0 mm or more, preferably 1.5 mm or more, more preferably 2.0 mm or more, even more preferably 2.5 mm or more, and particularly preferably 3.0 mm or more. The thickness may also be 10 mm or less, 8 mm or less, 6 mm or less, or 5 mm or less.

The width and length of the plate can be appropriately determined by factors such as the amount of feces sandwiched between two plates. For example, the length and width may be the same (that is, a square) or different (that is, a rectangle). An optional size may be employed for each of the length and width of the plate, and the length and width may also be, for example, preferably 5 mm or more, preferably 20 mm or more, and more preferably 30 mm or more. The length and width of the plate may each be 1,000 mm or less, 500 mm or less, 250 mm or less, 150 mm or less, or 100 mm or less.

The thickness of the feces sandwiched between two plates (holding surfaces) is not particularly limited as long as the feces can be dried (for example, as long as moving of water (water adsorption) from the inside of the feces to the plate is not inhibited). The thickness is, for example, 30 mm or less, preferably 15 mm or less, more preferably 5 mm or less, even more preferably 4 mm or less, and particularly preferably 2 mm or less. From the viewpoint of, for example, storing a large amount of feces by a plate having a length and width as short as possible, and recovering feces having an appropriate thickness (improving the recovering efficiency of dried feces), the thickness of feces to be sandwiched is, for example, 0.1 mm or more, preferably 1.0 mm or more, more preferably 1.5 mm or more, even more preferably 1.7 mm or more, and particularly preferably 2.0 mm or more.

The preferred feces fixing article of the present invention as described above may further include (c) a spacer between the first and second holding surfaces disposed facing each other from the viewpoint of, for example, strictly controlling the thickness of feces sandwiched between two plates (holding surfaces). The spacer may be designed so as to have a thickness corresponding to a desired thickness of feces. Accordingly, the spacer may have the dimension described above for the thickness of feces. Also, as the spacer, a plurality of spacers (for example, 2 to 4) may also be used. For example, in a case where two spacers are used, the spacers may also be sandwiched by two plates (holding surfaces) on both sides of feces sandwiched. In a case where three spacers are used, the spacers may also be sandwiched by two plates (holding surfaces) at positions on an equilateral triangle centered on the feces sandwiched. In a case where four spacers are used, the spacers may also be sandwiched by two plates (holding surfaces) at four sides along the length and width of the two plates (holding surfaces). Such use of four spacers enables isolation of feces from the outer environment besides strict control of the thickness of feces.

## Examples

Hereinafter, the present invention will be described in more detail with reference to Examples. However, the present invention is not limited to these Examples.

(Material)

(1) Water absorbing desiccant

(a) Desiccant (Power Dry Ultra X)

The desiccant was obtained from Daybreak Trading.

This desiccant is a water absorbing desiccant containing an inactive water absorbing polymer and calcium chloride (CaCl) as a component.

(b) Particulate silica gel

The particulate silica gel was obtained from Kanto Kagaku.

Silica gel Neo BLUE (registered trademark) (medium particle) 500 g Cat. No. 37489-08)

(c) Plate-like silica gel

Toyota plate, non-woven fabric packaged type, TPF3040, 30 mm x 40 mm, thickness: 3.2 mm

Toyota plate, unpackaged type, TP6040, 60 mm x 40 mm, thickness: 3.2 mm

Toyota plate, unpackaged type, TP3040, 30 mm x 40mm, thickness: 3.2 mm

Toyota plate, unpackaged type, TP3040, 30 mm x 40mm, thickness: 5 mm

(2) Feces sampling container "spoon type (containing a storage solution)"

The feces sampling container was obtained from TechnoSuruga Laboratory Co., Ltd.

Sample feces

In the following experiments, one human feces sample was divided into three and used for one condition (that is, n = 3). The water content of human feces is normally approximately 70 to 85% by weight.

Example 1: sample feces drying test using particulate silica gel

1-1. Study of drying condition in the case of using Power Dry

An experiment was conducted as follows.

(1) Sample feces were weighed in two 30 mL tubes (labcon).
(2) Each sample feces was accommodated in the 30 mL tube (labcon) with 1.27 g of Power Dry sprinkled on the sample feces, and allowed to stand at room temperature (25°C). The Power Dry and sample feces were sealed in the same closed space (30 mL tube) under a contact state.
(3) The tube was opened after approximately 20 hours, and the state of the sample feces were observed.

As a result, the sample feces became hard, and thus it was presumed that water was removed under a contact state. The degree of hardness of the sample feces was such that a significant force was required to crush the feces with a metal spatula. Thus, it was verified that contact of the sample feces with the water absorbing solid desiccant was effective for drying the sample feces.

1-2. Study of drying method using particulate silica gel

An experiment was conducted as follows.

(1) Particulate silica gel was placed in a 30 mL tube (labcon) and evenly spread, then sample feces that have been crushed and flattened were placed onto the particulate silica gel, and particulate silica gel was filled onto the sample feces at last (D3). That is, in D3, the sample feces that have been crushed and flattened are embedded in the particulate silica gel under the contact state with the particulate silica gel.
(2) Power Dry was placed in a 30 mL tube (labcon) and evenly spread, then sample feces that have been crushed and flattened were placed onto the Power Dry, and Power Dry was filled onto the sample feces at last (D4). That is, in D4, sample feces that have been crushed and flattened are embedded in the Power Dry under the contact state with the Power Dry.
(3) The cap of the 30 mL tube was covered with a Parafilm and this was allowed to stand at room temperature for approximately 40 hours.
(4) The 30 mL tube was opened and the sample feces were observed.

As a result, in D3 (contact state with particulate silica gel), dehydration of the sample feces progressed, and the sample feces changed like a dried tangle weed. On the other hand, in D4 (contact state with Power Dry), a portion that has been in contact with the desiccant was well dehydrated in the sample feces, but water was appeared to remain inside the sample feces. It was therefore verified that, in drying of the sample feces, silica gel was preferable as a water absorbing solid desiccant compared to Power Dry.

1-3. Study of effect of the weight ratio of particulate silica gel and feces and change over time

An experiment was conducted as follows.

(1) Approximately 1 g of sample feces was weighed into a 30 mL tube (labcon).
(2) Particulate silica gels were placed in amounts of, approximately, twice, three times, and six times the weight of the sample feces, and allowed to stand for 3, 6, 12, and 24 hours at room temperature (25°C) for respective amounts.
(3) After each 30 mL tube was opened, only the sample feces were taken out, and the weight of the sample feces was measured.
(4) The drying rate for each case was calculated assuming that "drying rate (%) = 100 × (weight before drying - weight after drying)/weight before drying" and the drying rates were compared.

As a result, it was found that, for the drying rate (%) after 24 hours, the case with a larger amount of silica gel results in higher final drying rate (the case of the weight of 6 times results in approximately 83% of drying rate). In particular, when silica gel in an amount six times the weight of feces was used, the feces were remarkably dried for approximately 6 hours (approximately 76%) .

1-4. Crushing of feces that have been dried using particulate silica gel

A method has been used in which lyophilizates of sample feces are crushed with beads in an operation such as extraction of metabolite(s) from sample feces (Example 2-1-1 in WO 2017/043087 A). Thus, whether crushing of non-lyophilized sample feces with beads is possible was studied.
An experiment was conducted as follows.

(1) Sample feces were sufficiently dried using particulate silica gel.
(2) Hard sample feces, which have been sufficiently dried, were crushed with beads. More specifically, sample feces that have been taken out and four zirconia beads (TOMY) each having a diameter (φ) of 3 mm were sealed in a 2 mL crushing tube. The sample feces were crushed by shaking using Shake master NEO (available from Bio-Medical Science Co., Ltd.) under the condition of 1,500 rpm for 10 min.
(3) The cap of the crushing tube was opened and the content was observed.

As a result, the sample feces could be crushed into a powder like state to some extent. It was therefore considered that this method was effective as a crushing method of the non-lyophilized sample feces.
However, in this method, residual of a mass of 1 to 2 mm was confirmed after crushing the sample feces. Although powder-like sample feces in an amount required for the analysis is considered to be obtained by this method, it was conceived that there was room for improvement from the viewpoint of obtaining uniform analysis sample.
Example 2: study of effect of drying and storage condition of sample feces on result of analysis of intestinal microbiota
DNA was extracted from sample feces dried and stored at normal temperature by various methods. The results of analysis of intestinal microbiota were compared with the existing method (Non-Patent Literature 1).

Sampling condition

A single sample feces (feces sampled in one-time defecation) were uniformly mixed, and respective treatments were performed based on the following different conditions.

[Table 1]

Table 1. List of sampling condition

| Sample No. | Sample name | Treatment method |
|---|---|---|
| 01 | Lyophilization after freezing at -80°C (1 day) | Stored at -80°C immediately after sampling, lyophilized 1 day after, and extracted DNA |
| 02 | Lyophilization after freezing at -80°C (5 days) | Stored at -80°C immediately after sampling, lyophilized 5 days after, and extracted DNA |
| 03 | Lyophilization after freezing with liquid nitrogen | Frozen with liquid nitrogen immediately after sampling, lyophilized immediately after freezing, and extracted DNA |
| 04 | Normal temperature (1 day) | Allowed to stand at normal temperature (25°C) for 1 day, lyophilized, and extracted DNA |

(continued)

| Sample No. | Sample name | Treatment method |
|---|---|---|
| 05 | Normal temperature (5 days) | Allowed to stand at normal temperature (25°C) for 5 days, lyophilized, and extracted DNA |
| 06 | RNA stabilization reagent (1 day) | Treated with RNA stabilization reagent immediately after sampling, allowed to stand at normal temperature (25°C) for 1 day, and extracted DNA |
| 07 | RNA stabilization reagent (5 days) | Treated with RNA stabilization reagent immediately after sampling, allowed to stand at normal temperature (25°C) for 5 days, and extracted DNA |
| 08 | Guanidine salt-containing storage solution (1 day) | Sampled in commercially available liquid immersion type container, allowed to stand in stabilization solution for 1 day, and extracted DNA |
| 09 | Guanidine salt-containing storage solution (5 days) | Sampled in commercially available liquid immersion type container, allowed to stand in stabilization solution for 5 days, and extracted DNA |
| 10 | Silica gel (1 day) | Dry-treated using particulate silica gel in amount three times amount of sample feces for 1 day, and extracted DNA |
| 11 | Silica gel (5 days) | Dry-treated using particulate silica gel in amount three times amount of sample feces for 5 days, and extracted DNA |
| Note<br>(1) As "RNA stabilization reagent", 1 ml of RNAlater (trade mark) Stabilization Solution (available from Thermo Fisher SCIENTIFIC) was used relative to 100 mg of sample feces.<br>(2) As "guanidine salt-containing storage solution", 1 ml of stabilization solution (guanidine thiocyanate-containing solution) contained in feces sampling container "spoon type (containing storage solution)" available from TechnoSuruga Laboratory Co., Ltd. was used relative to 100 mg of sample feces.<br>(3) As "silica gel", particulate silica gel (available from Kanto Kagaku) was used. | | |

Intestinal microbiota was analyzed by performing DNA extraction, preparation of a 16S rRNA library, and sequence analysis thereof in the same manner as in the method described in the previous report (Non-Patent Literature 1).

As a result, the ratios of Bacteroides, Blautia, Prevotella, and Barnesiellaceae significantly varied depending on the storage condition (Fig. 1). On the other hand, the ratios of microorganisms belonging to Faecalibacterium, Ruminococcaceae, Lachnospiraceae, Clostridiales, Parabacteroides, and Ruminococcus did not significantly vary depending on the storage condition (Fig. 1). The analysis result of intestinal microbiota of normal-temperature storage of sample feces using silica gel showed almost no change compared to the result of "lyophilization after freezing at -80°C" (5 days) (conventional condition: lyophilization described in Non-Patent Literature 1) (Fig. 2). The microorganisms whose ratios did not vary depending on the storage condition when considering the relationship of order, family, and genus were all microorganisms belonging to Clostridiales and Parabacteroides. Accordingly, it is conceived that the analysis of such microorganisms becomes a highly reliable indicator that does not depend on the drying method.

Also, as the one or more types of intestinal microbes whose existing ratios change in non-cryopreservation methods other than the storage method of the present invention (for example, storage at normal temperature or storage using an RNA stabilization reagent) compared to the conventional cryopreservation method (lyophilization), but can be secured in the storage method of the present invention at a level equivalent to that of the conventional cryopreservation method, microorganism belonging to Firmicutes, Bacteroidetes, Proteobacteria, Actinobacteria, and Verrucomicrobia were found (Figs.1 and 2). As a result of more specific analysis, it was confirmed that, as the microorganisms belonging to the above-described division, microorganisms belonging to at least Bacteroides, Blautia, Prevotella, Ruminococcus, and Barnesiellaceae existed (Fig. 1).

Also, comparing the intestinal microbiota profiles by using Weighted UniFrac distance, the condition that becomes a profile most similar to the conventional condition (the same as described above) was the condition of "liquid nitrogen (dried)". Among the conditions of storing at normal temperature, the condition of "silica gel (1 day)" was the most similar profile, and a similar profile was obtained in "silica gel (5 day)" (Fig. 3).

As described above, it was suggested that normal-temperature storage of sample feces using silica gel was a favorable method equivalent to the conventional method (Non-Patent Literature 1) even though it was performed at normal temperature, and such a normal-temperature storage did not affect the analysis result of intestinal microbiota.

Example 3: study of effect of drying and storage condition of sample feces on analysis result of metabolite(s) of intestinal microbiota

Among the conditions described in Table 1, metabolite extraction was performed for the sample numbers 01 to 08, 13, and 14 in the same manner as in the method described in WO 2017/043087 A. Further, to compare the difference in the concentration of the metabolite(s) between the sample number 02 "lyophilization after freezing at -80°C (5 days)" (conventional condition) and the sample number 14 "silica gel (5 days)" (condition of the present invention), the concentrations of respective metabolite(s) in the two conditions were shown by a scatter plot (Fig. 4).

As a result, among 513 types of metabolite(s) to be detected, 103 types were detected in "lyophilization after freezing at -80°C (5 days)" (conventional condition), and 108 types were detected in "silica gel (5 days)" (condition of the present invention). Among these metabolite(s), metabolite(s) that have been detected in only the conventional condition were 18 types, metabolite(s) that have been detected in only the condition of the present invention were 23 types, and metabolite(s) that have been detected in both conditions were 85 types.

Next, for 85 types of metabolite(s) detected in both "lyophilization after freezing at -80°C (5 days)" (conventional condition), and "silica gel (5 days)" (condition of the present invention), a variation ratio of mol number [nmol/g] per 1 g of sample feces (dried weight) was determined in accordance with Equation (1). Thus, the change in type of the metabolite(s) and change in level of the metabolite(s) when silica gel was used were calculated.

(Variation ratio) = (silica gel (5 days))/(lyophilization after freezing at -80°C (5 days))

Equation (1)

The result of classification based on the variation ratio is as follows.

[Table 2]

Table 2. Metabolites with significant amount decrease (variation ratio ≤ 0.3)

| No. | Metabolite name | Variation ratio |
|---|---|---|
| 1 | Adenosine monophosphate (AMP) | 0.25 |
| 2 | Arginine (Arg) | 0.04 |
| 3 | Glutamine (Gln) | 0.13 |
| 4 | Histidine (His) | 0.16 |
| 5 | Quinate | 0.28 |
| 6 | Threonine | 0.19 |

[Table 3-1]

Table 3-1. Metabolites with small amount variation (0.3 ≤ variation ratio < 3)

| No. | Metabolite name | Variation ratio |
|---|---|---|
| 1 | 2-Hydroxyglutarate | 1.60 |
| 2 | 2-Hydroxypentanoate | 0.70 |
| 3 | 2-Aminobenzamide: 2AB | 2.91 |
| 4 | 3-Methylbutanoate | 0.87 |
| 5 | 4-(β-acetylaminoethyl)imidazole | 1.02 |

(continued)

| No. | Metabolite name | Variation ratio |
|---|---|---|
| 6 | 4-Methyl-5-thiazoleetanol | 2.82 |
| 7 | 5-Aminovalerate | 2.83 |
| 8 | 7-Methylguanine | 0.49 |
| 9 | Adipate | 1.26 |
| 10 | Alanyl alanine (Ala-Ala) | 0.58 |
| 11 | α-aminoadipate | 0.67 |
| 12 | Anserine | 0.96 |
| 13 | Aspartate (Asp) | 0.38 |
| 14 | Azelate | 0.90 |
| 15 | Butyrate | 0.86 |
| 16 | Carnitine | 0.85 |
| 17 | Cholate | 0.54 |
| 18 | Choline | 1.00 |
| 19 | Citramalate | 0.78 |
| 20 | Citrate | 1.79 |
| 21 | Citrulline | 1.27 |
| 22 | Deoxycholate (DCA) | 0.72 |
| 23 | Diethanolamine | 1.75 |
| 24 | Dodecanedioate | 0.87 |
| 25 | γ-butyrobetaine | 0.69 |
| 26 | Glutamate (Glu) | 0.49 |
| 27 | Glutarate | 0.79 |
| 28 | Glycyl leucine (Gly-Leu) | 2.05 |
| 29 | Glycerophosphate | 0.39 |
| 30 | Glycolate | 2.80 |
| 31 | Guanine | 0.74 |
| 32 | Hexanoate | 0.80 |
| 33 | Histamine | 0.47 |

[Table 3-2]

Table 3-2. Metabolites with small amount variation (0.3 ≤ variation ratio < 3)

| No. | Metabolite name | Variation ratio |
|---|---|---|
| 34 | Homoserine | 1.55 |
| 35 | Hypoxanthine | 2.19 |
| 36 | Imiadzole-4-acetate | 0.81 |
| 37 | Isopropanolamine | 1.91 |

(continued)

| No. | Metabolite name | Variation ratio |
|---|---|---|
| 38 | Lactate | 1.16 |
| 39 | Lysine (Lys) | 0.51 |
| 40 | Malate | 2.53 |
| 41 | Malonate | 1.73 |
| 42 | Methionine (Met) | 2.12 |
| 43 | N-acetylaspartate | 0.80 |
| 44 | N-acetylglutamate | 1.38 |
| 45 | N-methylalanine | 0.95 |
| 46 | N-methylglutamate | 0.80 |
| 47 | N1-acetylspermidine | 1.41 |
| 48 | N1, N8-diacetylspermidine | 2.85 |
| 49 | Ornithine | 1.46 |
| 50 | Pelargonate | 0.76 |
| 51 | Pentanoate | 0.85 |
| 52 | Phthalate | 0.83 |
| 53 | Pimelate | 0.82 |
| 54 | Pipecolate | 0.86 |
| 55 | Proline (Pro) | 0.66 |
| 56 | Proline betaine | 0.40 |
| 57 | Propionate | 0.74 |
| 58 | Pyridoxamine | 2.38 |
| 59 | Sebacate | 0.79 |
| 60 | Serine (Ser) | 0.89 |
| 61 | Succinate | 1.72 |
| 62 | Terephthalate | 0.90 |
| 63 | Thiamine | 1.45 |
| 64 | Trigonelline | 0.81 |
| 65 | Trimethylamine | 0.35 |
| 66 | Tryptophan (Trp) | 2.05 |

[Table 4]

Table 4. Metabolites with significant amount increase (3 < variation ratio)

| No. | Metabolite name | Variation ratio |
|---|---|---|
| 1 | 3-Aminoisobutyrate | 3.04 |
| 2 | 5-Oxoproline | 24.30 |
| 3 | Alanine (Ala) | 4.25 |

(continued)

| No. | Metabolite name | Variation ratio |
|---|---|---|
| 4 | β-alanine (β-Ala) | 3.06 |
| 5 | γ-aminobutyrate (GABA) | 38.35 |
| 6 | Glycine (Gly) | 4.21 |
| 7 | Isoleucine (Ile) | 11.11 |
| 8 | Leucine (Leu) | 15.73 |
| 9 | N-acetylornithine | 3.34 |
| 10 | Phenylalanine (Phe) | 5.97 |
| 11 | Putrescine (1,4-butane diamine) | 4.58 |
| 12 | Tyrosine (Tyr) | 3.56 |
| 13 | Valine (Val) | 6.92 |

From the result, metabolite(s) with a small variation in amount regardless of the drying method were found as shown in Tables 3-1 and 3-2. It should be noted that, as the group of metabolite(s) with a small variation in amount, short-chain fatty acids [for example, propionate, and butyrate], bile acids [for example, cholate, and deoxycholate (DCA)], and trimethylamine were found. These metabolite(s) can be an important indicator for considering the relationship between health and the intestinal bacteria. Accordingly, it can be conceived that the quantitative analysis of metabolite(s) shown in Tables 3-1 and 3-2 is not only a highly reliable indicator that does not depend on the drying method, but also can be an important indicator for considering the relationship between health and the intestinal bacteria.

Note that, considering a reason why metabolite(s) whose amounts increase or decrease in "lyophilization after freezing at -80°C (5 days)" (conventional condition) and "silica gel (5 days)"(condition of the present invention), existence of such metabolite(s) may be caused by water in the form of liquid that is required for metabolic reaction of many bacteria. In the conventional condition, feces are frozen immediately after sampling, and water is removed by lyophilization in drying. Thus, time at which water in the form of liquid exists until the analysis is very short. In contrast, in the drying with silica gel, the time at which water in the form of liquid exists is considered to become longer compared to the conventional condition. Thus, a certain period of time is required until the metabolism of bacteria is completely stopped. Accordingly, it is conceived that since metabolite(s), which are produced or consumed due to bacteria in a period during which water in the form of liquid, exists, both metabolite(s) which decrease and increase compared to conventional condition exist.

Example 4: feces drying test using plate-like silica gel

4-1. Purpose

Examples 1 to 3 show that removal of water is effective for stable storage of DNA and metabolite(s) in sample feces. Although it was verified in the process of the experiment that the contact between the sample feces and particulate silica gel was effective for sufficient drying, the sample feces after drying adhered to the particulate silica gel, and thus separation thereof was complicated. In addition, considering practical use of this method, an operation of adhering the particulate silica gel to the sample feces is not easy in handling because the particulate silica gel may drop from the container, for example.

Thus, the inventors focused on a plate-like silica gel obtained by forming silica gel fine powder. Unlike the particulate silica gel that requires a large amount for embedding a mass of sample feces, the plate-like silica gel with a sufficient size allows contact with only two sheets of top and bottom plate-like silica gels for the sample feces. Further, since the plate-like silica gel has a smooth surface, it can be expected that sample feces can be easily teared off from silica gel after drying the sample.

4-2. Drying of sample feces using plate-like silica gel (non-woven fabric packaged type)

An experiment was conducted as follows.

[1] Approximately 1 g of sample feces was placed on a plate-like silica gel (non-woven fabric packaged type), and pressed with another plate-like silica gel so as to entirely spread the sample feces, and this was allowed to stand at room temperature (25°C) for approximately 6 hours.

[2] The sample which has been allowed to stand was separated, and then the sample feces were observed.

As a result, the sample feces were not dried at all. From this result, it was presumed that the feces and the silica gel main body did not come into contact due to the surface of the silica gel being covered with the non-woven fabric, and as a result, drying was prevented. Thus, a desiccant to be used was reviewed. In addition, since it was suggested that contact with silica gel was effective for drying the sample feces, use of silica gel with no non-woven fabric was studied.

4-3. Drying of sample feces using plate-like silica gel (unpackaged type)

An experiment was conducted as follows.

[1] Approximately 1 g of sample feces was placed on a plate-like silica gel, and pressed with another plate-like silica gel so as to entirely spread the sample feces, and this was allowed to stand at room temperature (25°C). Two sets of the above samples were prepared.

[2] For one of the sets prepared in the above-described step [1], the sample was separated after approximately 2 hours, and the sample feces were observed.

[3] For the rest of the sets prepared in the above-described step [1], the sample was separated after approximately 6 hours and observed.

Note that, from the silica gel separated after drying, it was presumed that the contact area between the sample feces and silica gel in one surface of the plate-like silica gel to which the feces adhered was approximately 1,350 $mm^2$. In consideration that feces were similarly in contact with both surfaces of the plate-like silica gel, the contact area in a case where approximately 1 g of sample feces was sandwiched between two plates with a size of 60 mm x 40 mm was estimated to be approximately 2,700 $mm^2$.

As a result, handling of the plate-like silica gel used was the easiest among the materials that have been used so far. Also, the sample feces was sufficiently dried after elapse of 6 hours. For the particulate silica gel, an operation of removing silica gel from the dried feces was difficult, but for the plate-like silica gel, separation from the sample feces was easily made by scraping with a metal spatula.

Hereinafter, a drying condition of the sample feces in a case of using the plate-like silica gel was studied.

4-4. Study of feces to be used as sample for dehydration experiment

The sample feces drying experiment using the plate-like silica gel was repeatedly conducted. Considering that sample feces can be stably supplied if the sample feces that have been frozen once and dissolved are dealt similarly to the unfrozen sample feces, unfrozen feces and freeze-thawed feces were each dried with the plate-like silica gel, and the weight change of each sample over time was checked.

An experiment was conducted as follows.

[1] Approximately 1 g of sample feces was weighed into a feces sampling tube in advance, and lyophilized.

The proportion of water contained in feces was calculated by measuring the weight before and after lyophilization.

[2] Sample feces that are the same as the sample feces used in the above-described step [1] were weighed so as to be approximately 1 g using a spatula and a measuring cup.

[3] The weighed feces were placed on the proximity of a central portion of one sheet of plate-like silica gel (30 mm x 40 mm x 3.2 mm), and the shape of the feces were arranged so as to conform to the shape of the plate-like silica gel with a spatula.

[4] Two pieces of plastic with a height of 2 mm as a spacer were disposed around the feces.

[5] Another plate-like silica gel was placed on the top of the feces to sandwich the feces, and the plate-like silica gel was slightly pressed by hand from upward so that the thickness of the feces is equivalent to the thickness of the spacer.

[6] The feces sandwiched with plate-like silica gels were placed in a zippered plastic bag (UNIPACK (available from Seisannipponsha Ltd., E-8)), and air was removed as much as possible to seal the bag.

[7] The feces were allowed to stand at room temperature (25°C) for 10, 30, 60, 90, and 120 minutes.

One sample was prepared for each condition and treatment time, that is, a total of ten samples were prepared and each bag was opened at every time point.

[8] Each UNIPACK (available from Seisannipponsha Ltd., E-8) was opened and the sample was separated. Then, the feces were scraped off into the measuring cup in a manner that the feces were scraped off from the silica gel by using a flat side of the spatula.

[9] The weight of the scraped feces was measured and recorded. The water content after the silica gel treatment was determined based on the mass of water lost by (a) lyophilizing the sample after silica gel treatment and determining the mass of water lost from the weight change before and after lyophilization.

As a result, a great difference in drying behavior was not confirmed between unfrozen feces and freeze-thawed feces (Table 5 and Fig. 5). Accordingly, freeze-thawed feces were used in the following experiments in consideration of convenience properties. Further, it was found that standing at room temperature for 90 minutes or more was effective to achieve the water content of less than 10%.

[Table 5]

Table 5. Comparison of water contents of unfrozen feces and freeze-thawed feces relative to drying time (25°C)

| Time course (/min) | Water content (/%) | |
|---|---|---|
| | Unfrozen feces | Freeze-thawed feces |
| 0 | 77.1 | 77.1 |
| 10 | 53.0 | 53.7 |
| 30 | 39.3 | 32.8 |
| 60 | 13.2 | 12.5 |
| 90 | 7.8 | 2.3 |
| 120 | 6.7 | 3.1 |

4-5. Drying behavior at low temperature (4°C)

When sample feces were treated with plate-like silica gel, drying behavior under a low temperature condition (4°C) was evaluated over time.

An experiment was conducted as follows.

[1] An experimental operation similar to that of the section 4-4 was performed (excluding step [7]).
[2] Feces were allowed to stand in a refrigerator (4°C) in step [7]. Also, samples were allowed to stand for 10, 30, 60, 90, 120, 150, 180, 300, 540, and 720 minutes. One sample was prepared for each treatment time, that is, a total of ten samples were prepared.

The result showed that the water content in the sample feces fell below 10% in standing for 540 minutes, and drying was delayed compared to drying at normal temperature (25°C) that required 90 minutes (Table 6 and Fig. 6). In addition, the final water content was 3.1% at normal temperature (25°C), whereas the final water content was 6.6 %, which was a high value, at 4°C (Table 6 and Fig. 6). Thus, it was found that the case where silica gel was used at a low temperature condition requires time for decreasing the water content.

[Table 6]

Table 6. Transition of water content of sample feces relative to drying time (4°C)

| Time course (/min) | Water content (/%) |
|---|---|
| 0 | 77.0 |
| 10 | 72.0 |
| 30 | 64.5 |
| 60 | 54.3 |
| 90 | 47.0 |
| 120 | 41.0 |
| 150 | 33.7 |
| 180 | 29.6 |

(continued)

| Time course (/min) | Water content (/%) |
|---|---|
| 300 | 14.5 |
| 540 | 6.6 |

4-6. Drying behavior at high temperature (50°C or higher)

When sample feces were treated with plate-like silica gel, drying behavior under a high temperature condition (50°C or higher) was evaluated over time. As a method of easily reproducing the experimental temperature range, a method of heating a sample with a body warmer (available from Wing corporation, "Atatamaru Kairo" stick-on-type, 10 bags) attached to the outer surface of the UNIPACK (available from Seisannipponsha Ltd., E-8) was employed.

An experiment was conducted as follows.

[1] Sample feces were weighed into a feces sampling tube in advance, and lyophilized.
The proportion of water contained in feces was calculated by measuring the weight before and after lyophilization.
[2] Body warmers were attached to the front and back surfaces of the UNIPACK (available from Seisannipponsha Ltd., E-8), and the change over time of the internal temperature was recorded using a digital thermometer.
[3] It was confirmed that the temperature reached 50°C, which was the temperature that can be maintained as shown in the product specification of the body warmer.
[4] Sample feces that are the same as the sample feces used in the above-described step [1] were weighed so as to be approximately 1 g using a spatula and a measuring cup.
[5] The weighed feces were placed on the proximity of a central portion of one sheet of plate-like silica gel (30 mm x 40 mm x 3.2 mm), and the shape of the feces were arranged so as to conform to the shape of the plate-like silica gel with a spatula.
[6] Two pieces of spacers (height: 2 mm) were disposed around the feces.
[7] Another plate-like silica gel was placed on the top of the feces to sandwich the feces, and the plate-like silica gel was slightly pressed so that the height of the feces reaches the height of the spacers.
[8] The feces sandwiched with plate-like silica gels were placed in the UNIPACK (available from Seisannipponsha Ltd., E-8)), and air was removed as much as possible to seal the bag.
[9] A piece of plate-like expanded polystyrene placed as a heat-shielding plate was used as a base. A Kim towel, the sample with a body warmer, a Kim towel, expanded polystyrene, an aluminum block were stacked on the base in this order, and this was allowed to stand at room temperature for 10, 30, 60, 90, and 120 minutes. One sample was prepared for each condition and treatment time, that is, a total of five samples were prepared, and each bag was opened at every time point.
[10] Each UNIPACK (available from Seisannipponsha Ltd., E-8) was opened and the sample was separated. Then, the feces were scraped off into the measuring cup in a manner that the feces are scraped off from the silica gel by using a flat side of the spatula.
[11] The weight of the scraped feces was measured and recorded.

The result showed that the internal temperature of the body warmer reached 50°C approximately 50 minutes after opening the body warmer, and the temperature was maintained at 50°C or higher until the maximum measurement time of 250 minutes (Table 7 and Fig. 7). Further, the temperature increase was continued until approximately 100 minutes later and the maximum temperature of 60.0°C (actual value) was recorded.

[Table 7]

Table 7. Change over time of temperature of sample feces when disposable body warmer is used

| Elapsed time (/min) | Measured temperature (°C) |
|---|---|
| 0 | 26.8 |
| 30 | 41.8 |
| 43 | 44.7 |
| 53 | 50.7 |
| 58 | 52.8 |

(continued)

| Elapsed time (/min) | Measured temperature (°C) |
|---|---|
| 73 | 57 |
| 90 | 60 |
| 135 | 60 |
| 143 | 58.7 |
| 170 | 56.1 |
| 180 | 53.7 |
| 185 | 54.1 |
| 250 | 53.9 |

Additionally, it was found that almost all the water in the sample feces was removed by heating using the body warmer and standing for 120 minutes or more (Table 8 and Fig. 8). It was also found that the water content in the sample feces falls below 10% in standing for 30 minutes, and more quick drying was possible than drying at normal temperature (25°C) that required 90 minutes (Table 8 and Fig. 8).

[Table 8]

Table 8. Change over time of water content of sample feces sandwiched between plate-like silica gel at high temperature condition (50°C or higher)

| Time course (/min) | Water content (/%) |
|---|---|
| 0 | 77.1 |
| 10 | 47.3 |
| 30 | 9.4 |
| 60 | 8.9 |
| 90 | 2.7 |
| 120 | 1.5 |

4-7. Study of drying method using microwave oven

It was conceived that the ratio of the microbiota and metabolite(s) could be fixed by instantaneously heating the sample feces to stop the biological activity of bacterial cells. Thus, among familiar devices, focusing on a microwave oven which heats water by electromagnetic waves, effects of combined use with drying with plate-like silica gel on the drying behavior was studied.

An experiment was conducted as follows.

[1] Sample feces were weighed into a feces sampling tube in advance, and lyophilized.
The proportion of water contained in feces was calculated by measuring the weight before and after lyophilization.
[2] Sample feces that are the same as the sample feces used in the above-described step [1] were weighed to approximately 1 g using a spatula and a measuring cup.
[3] The weighed feces were placed on the proximity of a central portion of one sheet of plate-like silica gel (30 mm x 40 mm x 3.2 mm), and the shape of the feces were arranged so as to conform to the shape of the plate-like silica gel with a spatula.
[4] Two pieces of spacers (height: 2 mm) were disposed around the feces.
[5] Another plate-like silica gel was placed on the top of the feces to sandwich the feces, and the plate-like silica gel was slightly pressed so that the height of the feces reaches the height of the spacers.
[6] The feces sandwiched with plate-like silica gels were placed in the UNIPACK (available from Seisannipponsha Ltd., E-8)), and air was removed as much as possible to seal the bag.
[7] The sample was allowed to stand at the center of the microwave oven and heated with an output power of 500 W for 10, 20, 30 seconds. One sample was prepared for each treatment time, that is, a total of three samples were prepared, and each bag was opened at every time point.
[8] Each UNIPACK (available from Seisannipponsha Ltd., E-8) was opened and the sample was separated. Then, the feces were scraped off into the measuring cup in a manner that the feces were scraped off from the silica gel by using a flat side of the spatula.
[9] The weight of the scraped feces was measured and recorded.

The result showed that water in the sample feces was rapidly removed by drying of the sample feces using the plate-like silica gel and microwave oven (500 W, 30 seconds) (Table 9 and Fig.9). It was found that the water content reached approximately 10% in the treatment time of 10 seconds, and fell below 10% in 20 seconds (Table 9 and Fig.9).

[Table 9]

Table 9. Change over time of water content of sample feces sandwiched between plate-like silica gel in case of heat-treating in microwave oven

| Time course (/min) | Water content (/%) |
|---|---|
| 0 | 77.1 |
| 10 | 10.4 |
| 20 | 7.0 |
| 30 | 4.3 |

4-8. Study of drying condition under anaerobic conditions (1)

It is known that fungi contained in feces include those requiring oxygen besides water in their metabolic activity. To prevent the metabolic activity by removing oxygen in the atmosphere, effects of presence of a deoxidizer on drying of sample feces using plate-like silica gel were firstly studied.
An experiment was conducted as follows.

[1] Sample feces were weighed into a feces sampling tube in advance, and lyophilized.
The proportion of water contained in feces was calculated by measuring the weight before and after lyophilization.
[2] Sample feces that are the same as the sample feces used in the above-described step [1] were weighed to approximately 1 g using a spatula and a measuring cup.
[3] The weighed feces were placed on the proximity of a central portion of one sheet of plate-like silica gel (30 mm x 40 mm x 3.2 mm), and the shape of the feces were arranged so as to conform to the shape of the plate-like silica gel with a spatula.
[4] Two pieces of spacers (height: 2 mm) were disposed around the feces.
[5] Another plate-like silica gel was placed on the top of the feces to sandwich the feces, and the plate-like silica gel was slightly pressed so that the height of the feces reaches the height of the spacers.
[6] Two sets of samples in the above-described steps [2] to [5] were prepared, and one of the samples was placed in Lamizip, stand type, with low oxygen permeability (available from Seisannipponsha Ltd., AL-9), and air was removed as much as possible to seal the bag. The other one was placed in Lamizip, stand type (available from Seisannipponsha Ltd., AL-9) with a deoxidizer (available from Torishige Sangyo Co., Ltd., Ever Fresh, QJ-30), and air was removed as much as possible to seal the bag.
[7] The sample sets were allowed to stand at room temperature (25°C) for 10, 30, 60, 90, and 120 minutes. One sample was prepared for each condition and treatment time, that is, a total of ten samples were prepared, and each bag was opened at every time point.
[8] Each Lamizip stand type was opened, and the sample was separated. Then, the feces were scraped off into the measuring cup in a manner that the feces are scraped off from the silica gel by using a flat side of the spatula.
[9] The weight of the scraped feces was measured and recorded.

As a result, a great difference in drying behavior was not confirmed between the case of enclosing the deoxidizer and the case of not enclosing the deoxidizer (Table 10 and Fig. 10). It was found that the time at which the water content fell below 10% also required standing for 90 minutes or more, similarly to the case of not enclosing the deoxidizer (Table 10 and Fig. 10).

[Table 10]

Table 10. Change over time of water content of sample feces sandwiched between plate-like silica gel at time of enclosing deoxidizer or not enclosing deoxidizer

| Time course (/min) | Water content (/%) | |
|---|---|---|
| | With deoxidizer | Without deoxidizer |
| 0 | 77.1 | 77.1 |
| 10 | 51.3 | 53.7 |
| 30 | 23.4 | 32.8 |
| 60 | 13.2 | 12.5 |
| 90 | 7.0 | 2.3 |
| 120 | 5.0 | 3.1 |

4-9. Study of drying method of using particulate silica gel and not bringing feces and silica gel into direct contact

It has been verified that contact of the feces and the plate-like silica gel allowed drying at room temperature. However, when actually dealing dried feces as a sample, the step of separating the feces from the plate-like silica gel is the rate-limiting step of the work. Thus, whether drying is possible by enclosing and sealing feces with silica gel was studied.

An experiment was conducted as follows.

[1] Approximately 2 g of dried particulate silica gel was placed in the bottom part of a sampling tube in which a rod-like sample collecting part is connected to a screw cap.
[2] A piece of ring-shaped paper tape, the inner surface of which has been coated with particulate silica gel was laminated on the sampling tube.
[3] Sample feces were weighed into a feces sampling tube in advance, and lyophilized. The proportion of water contained in feces was calculated by measuring the weight before and after lyophilization.
[4] The weight of the sampling tube containing the silica gel was measured in advance, and the weight of the sampled feces was calculated by measuring the weight before and after sampling.
[5] Sample feces that are the same as the sample feces used in the above-described step [3] were sampled with a sampling rod with a screw cap.
[6] The sampling rod was inserted so that stool(s) were not in contact with the silica gel on the wall surface of the sampling tube. The container was sealed and allowed to stand at room temperature (25°C) for 120 minutes.
[7] The sampling tube was opened, and the sample was recovered.
[8] The weight of the recovered feces was measured and recorded.

The result showed that, in a case where 100.5 mg of feces were sampled, the feces were dried to 31.2 mg (water content: 6.8%). It was found from this result that a desired degree of drying could be achieved even in a non-contact state with silica gel if the amount of feces sample is small.

Example 5: culture experiment of microorganisms contained in feces after drying

A culture experiment using stool(s) immediately after excretion, frozen stool(s), lyophilized feces, and feces dried with silica gel were conducted. The culture experiment was conducted as follows.

[1] Each of the sample feces was suspended in a phosphate buffer solution. At that time, the suspension was prepared so that the weights of components of the feces were equal. In other words, considering the water content of the feces, 20 mg of feces immediately after excretion, and 6 mg of feces dried with silica gel were suspended relative to 1 mL of phosphate buffer solutions.
[2] Each of the obtained suspensions was applied to a culture medium specialized for growth of Bifidobacterium (TOS propionate agar (Yakult Pharmaceutical Industry Co., Ltd.)), and allowed to stand at 37°C under anaerobic conditions using an aeropack overnight.
[3] Colonies of fungi generated on the culture medium were checked, and the number of colonies were counted.
[4] The culturing efficiency was determined. The culturing efficiency was calculated in accordance with the following

equation.

(Culturing efficiency) = (the number of colonies counted in each condition)/(the number of colonies counted in a case where feces immediately after excretion were used) x 100 (%)

[5] The culturing efficiency was determined as follows.

Feces immediately after excretion: 100%, frozen feces: 37.6%, lyophilized feces: 0.1%, and feces dried with silica gel: 15.6%.

As described above, it was found that the drying method using silica gel was effective as a storage method of microorganisms that directs utilization and application in addition to storage of genetic information and metabolite(s) in feces.

Industrial Applicability

For example, the present invention is effective for storage of feces and microorganisms contained in the feces and analysis thereof.

## Claims

**1.** A storage method of feces, the storage method comprising drying the feces in presence of a solid desiccant.

**2.** The storage method according to claim 1, wherein the feces are feces of animals, fishes or insects.

**3.** The storage method according to claim 1 or 2, wherein the solid desiccant is a water absorbing solid desiccant.

**4.** The storage method according to claim 3, wherein the water absorbing solid desiccant is silica gel.

**5.** The storage method according to any one of claims 1 to 4, wherein the drying of the feces is performed under a contact state with the solid desiccant, and
a contact area between the feces and the solid desiccant is 100 ($mm^2$/1 g of feces) or more.

**6.** The storage method according to any one of claims 1 to 4, wherein the drying of the feces is performed under a non-contact state with the solid desiccant, and
a weight ratio of the feces to the solid desiccant (solid desiccant/feces) is 0.1 to 10,000.

**7.** The storage method according to any one of claims 1 to 6, wherein the drying is performed at a temperature higher than 0°C.

**8.** The storage method according to claim 7, wherein the drying is performed at 4 to 60°C.

**9.** The storage method according to claim 7, wherein the drying is performed by heating using a heating device.

**10.** The storage method according to claim 9, wherein the heating device is a microwave irradiator.

**11.** An analysis method of feces, the method comprising:

(1) drying the feces in presence of a solid desiccant; and
(2) analyzing the dried feces.

**12.** The method according to claim 11, wherein the analysis is performed on an intestinal bacteria and/or substance in the feces.

13. The method according to claim 12, wherein the intestinal bacteria is one or more types of microorganisms selected from the group consisting of microorganisms belonging to Clostridiales and microorganisms belonging to Parabacteroides.

14. The method according to claim 12, wherein the intestinal bacteria is one or more types of microorganisms selected from the group consisting of Firmicutes, Bacteroidetes, Proteobacteria, Actinobacteria, and Verrucomicrobia.

15. The method according to claim 12, wherein the intestinal bacteria is one or more types of microorganisms selected from the group consisting of Bacteroides, Blautia, Prevotella, Ruminococcus, and Barnesiellaceae.

16. The method according to any one of claims 12 to 15, wherein the substance includes one or more types of substances selected from the group consisting of:

(1) 2-hydroxyglutarate;
(2) 2-hydroxypentanoate;
(3) 2-aminobenzamide;
(4) 3-methylbutanoate;
(5) 4-(β-acetylaminoethyl)imidazole;
(6) 4-methyl-5-thiazole ethanol;
(7) 5-aminopentanoate;
(8) 7-methylguanine;
(9) adipate;
(10) alanyl alanine;
(11) α-aminoadipate;
(12) anserine;
(13) aspartate;
(14) azelate;
(15) butyrate;
(16) carnitine;
(17) cholate;
(18) choline;
(19) citramalate;
(20) citrate;
(21) citrulline;
(22) deoxycholate;
(23) diethanolamine;
(24) dodecanedioate;
(25) γ-butyrobetaine;
(26) glutamate;
(27) glutarate;
(28) glycyl leucine;
(29) glycerophosphate;
(30) glycolate;
(31) guanine;
(32) hexanoate;
(33) histamine;
(34) homoserine;
(35) hypoxanthine;
(36) imiadzole-4-acetate;
(37) isopropanolamine;
(38) lactate;
(39) lysine;
(40) malate;
(41) malonate;
(42) methionine;
(43) N-acetylaspartate;

(44) N-acetylglutamate;
(45) N-methylalanine;
(46) N-methylglutamate;
(47) N1-acetylspermidine;
(48) N1, N8-diacetylspermidine
(49) ornithine
(50) pelargonate
(51) pentanoate;
(52) phthalate;
(53) pimelate;
(54) pipecolate;
(55) proline;
(56) proline betaine;
(57) propionate;
(58) pyridoxamine;
(59) sebacate;
(60) serine;
(61) succinate;
(62) terephthalate;
(63) thiamine;
(64) trigonelline;
(65) trimethylamine; and
(66) tryptophan.

**17.** A hermetic container comprising: (a) a solid desiccant; and (b) feces under a non-contact state with the solid desiccant.

**18.** The hermetic container according to claim 17, wherein a weight ratio of the feces to the solid desiccant (solid desiccant/feces) is 0.1 to 10,000.

**19.** The hermetic container according to claim 17 or 18, wherein the solid desiccant is a water absorbing solid desiccant.

**20.** The hermetic container according to claim 19, wherein the water absorbing solid desiccant is silica gel.

**21.** The hermetic container according to any one of claims 17 to 20, wherein the feces are dried.

**22.** A feces fixing article comprising: (a) one or more members containing or fixing a solid desiccant; and (b) feces under a contact state with the solid desiccant.

**23.** The feces fixing article according to claim 22, comprising: (a1) a first plate having a first holding surface; (a2) a second plate having a second holding surface; and (b) the feces, wherein
the feces are sandwiched between the first and second holding surfaces, which are disposed to face each other, to be in contact with the solid desiccant, and
a contact area between the feces and a holding surface containing the solid desiccant is 100 ($mm^2$/1 g of feces) or more.

**24.** The feces fixing article according to claim 22 or 23, further comprising (c) a spacer between the first and second holding surfaces disposed facing each other.

**25.** The feces fixing article according to any one of claims 22 to 24, wherein at least one of a first member and a second member is a water absorbing solid desiccant plate.

**26.** The feces fixing article according to claim 25, wherein the water absorbing solid desiccant plate is a silica gel plate.

**27.** The feces fixing article according to any one of claims 22 to 26, wherein the feces are dried.

FIG. 1

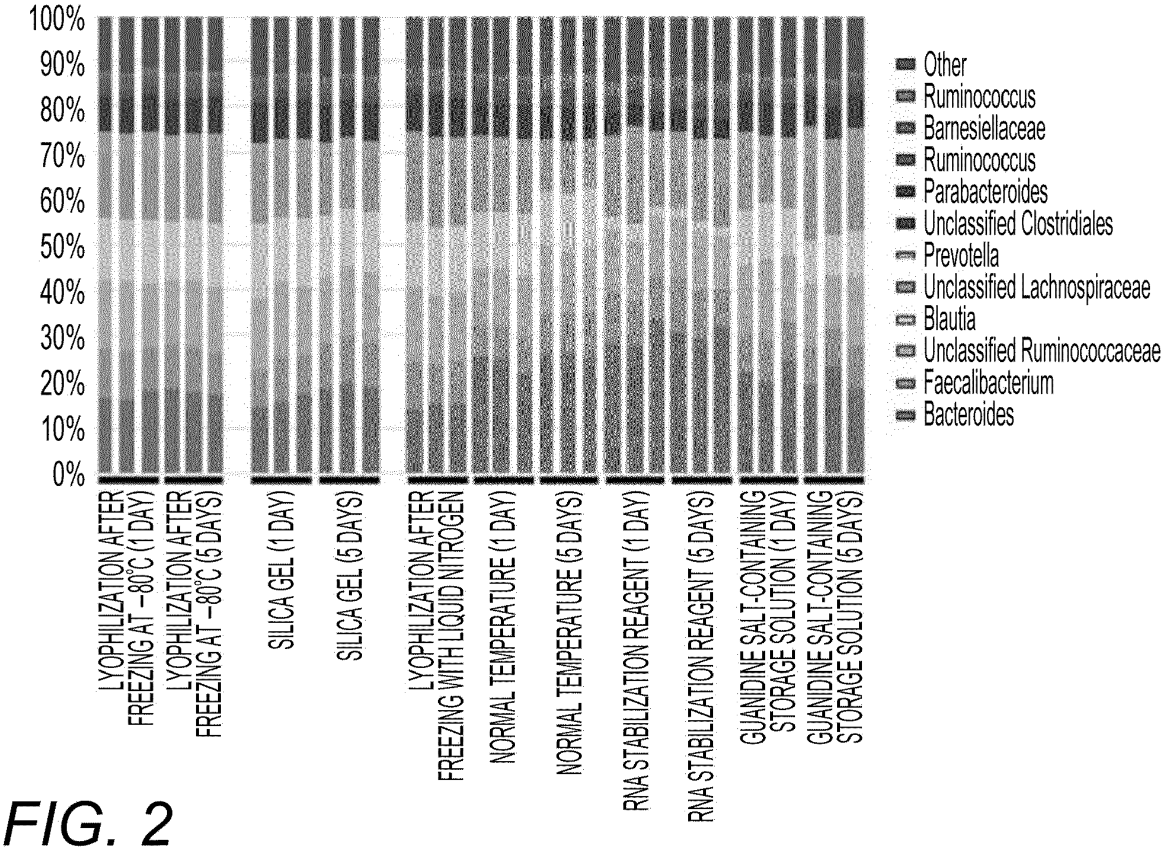
100%
90%
80%
70%
60%
50%
40%
30%
20%
10%
0%
Other
Ruminococcus
Barnesiellaceae
Ruminococcus
Parabacteroides
Unclassified Clostridiales
Prevotella
Unclassified Lachnospiraceae
Blautia
Unclassified Ruminococcaceae
Faecalibacterium
Bacteroides
LYOPHILIZATION AFTER FREEZING AT −80°C (1 DAY)
LYOPHILIZATION AFTER FREEZING AT −80°C (5 DAYS)
SILICA GEL (1 DAY)
SILICA GEL (5 DAYS)
LYOPHILIZATION AFTER FREEZING WITH LIQUID NITROGEN
NORMAL TEMPERATURE (1 DAY)
NORMAL TEMPERATURE (5 DAYS)
RNA STABILIZATION REAGENT (1 DAY)
RNA STABILIZATION REAGENT (5 DAYS)
GUANIDINE SALT-CONTAINING STORAGE SOLUTION (1 DAY)
GUANIDINE SALT-CONTAINING STORAGE SOLUTION (5 DAYS)

FIG. 2

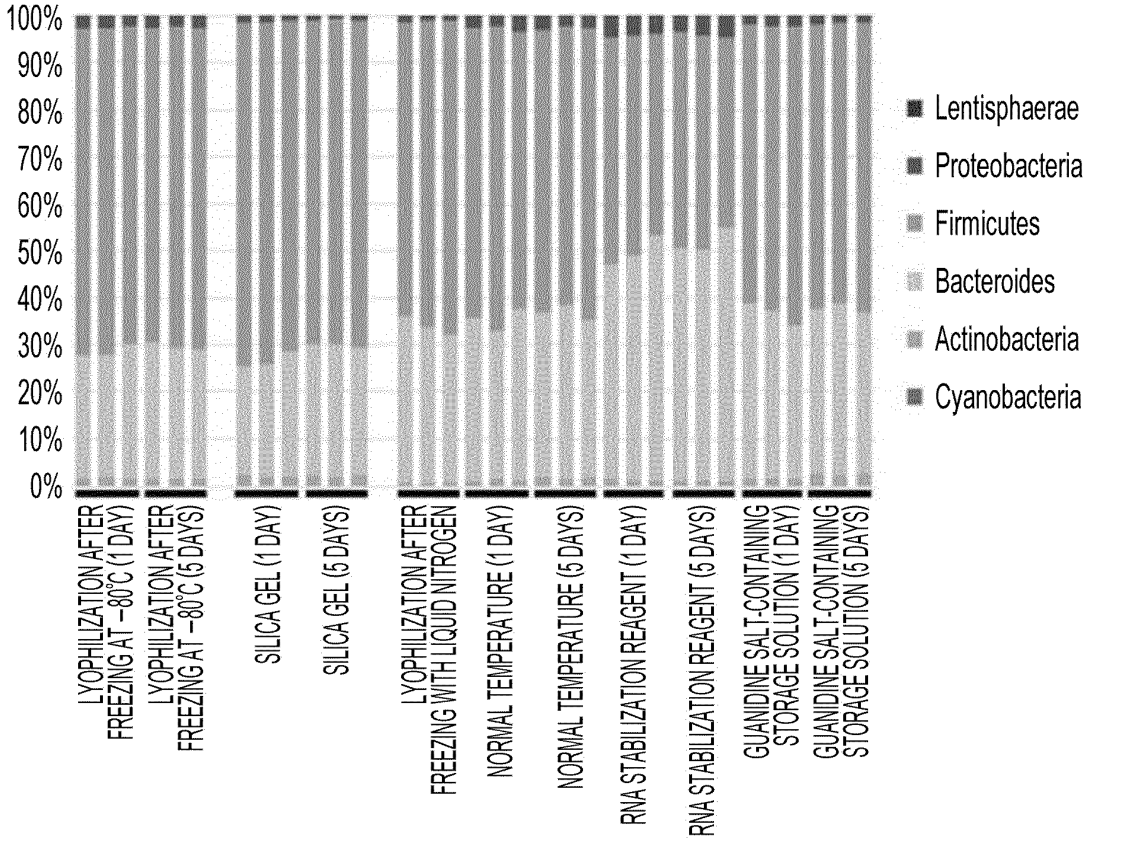
100%
90%
80%
70%
60%
50%
40%
30%
20%
10%
0%
Lentisphaerae
Proteobacteria
Firmicutes
Bacteroides
Actinobacteria
Cyanobacteria
LYOPHILIZATION AFTER FREEZING AT −80°C (1 DAY)
LYOPHILIZATION AFTER FREEZING AT −80°C (5 DAYS)
SILICA GEL (1 DAY)
SILICA GEL (5 DAYS)
LYOPHILIZATION AFTER FREEZING WITH LIQUID NITROGEN
NORMAL TEMPERATURE (1 DAY)
NORMAL TEMPERATURE (5 DAYS)
RNA STABILIZATION REAGENT (1 DAY)
RNA STABILIZATION REAGENT (5 DAYS)
GUANIDINE SALT-CONTAINING STORAGE SOLUTION (1 DAY)
GUANIDINE SALT-CONTAINING STORAGE SOLUTION (5 DAYS)

FIG. 3

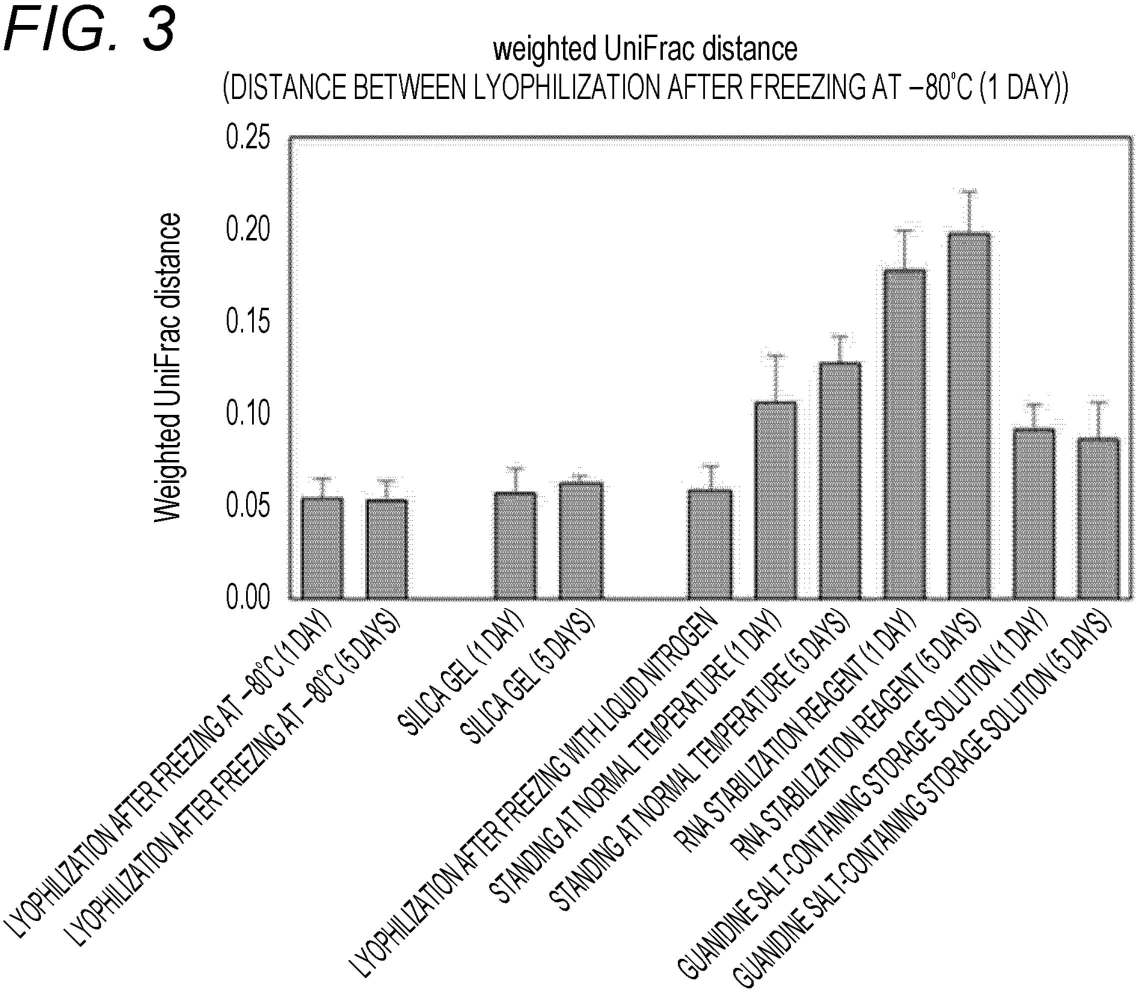
weighted UniFrac distance
(DISTANCE BETWEEN LYOPHILIZATION AFTER FREEZING AT −80°C (1 DAY))
Weighted UniFrac distance
0.25
0.20
0.15
0.10
0.05
0.00
LYOPHILIZATION AFTER FREEZING AT −80°C (1 DAY)
LYOPHILIZATION AFTER FREEZING AT −80°C (5 DAYS)
SILICA GEL (1 DAY)
SILICA GEL (5 DAYS)
LYOPHILIZATION AFTER FREEZING WITH LIQUID NITROGEN
STANDING AT NORMAL TEMPERATURE (1 DAY)
STANDING AT NORMAL TEMPERATURE (5 DAYS)
RNA STABILIZATION REAGENT (1 DAY)
RNA STABILIZATION REAGENT (5 DAYS)
GUANIDINE SALT-CONTAINING STORAGE SOLUTION (1 DAY)
GUANIDINE SALT-CONTAINING STORAGE SOLUTION (5 DAYS)

FIG. 4

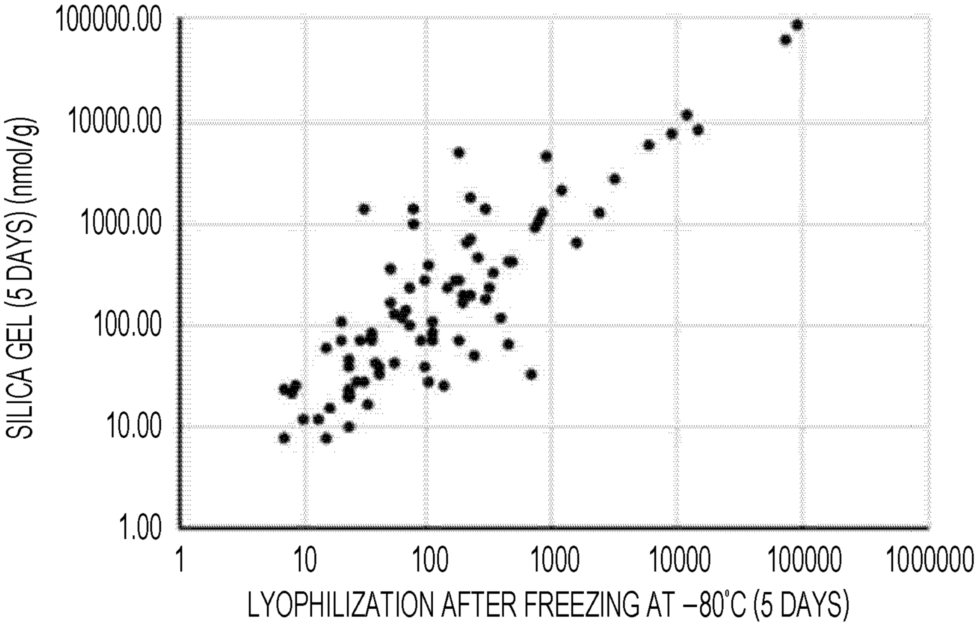
100000.00
10000.00
1000.00
100.00
10.00
1.00
SILICA GEL (5 DAYS) (nmol/g)
1
10
100
1000
10000
100000
1000000
LYOPHILIZATION AFTER FREEZING AT −80°C (5 DAYS)

FIG. 5

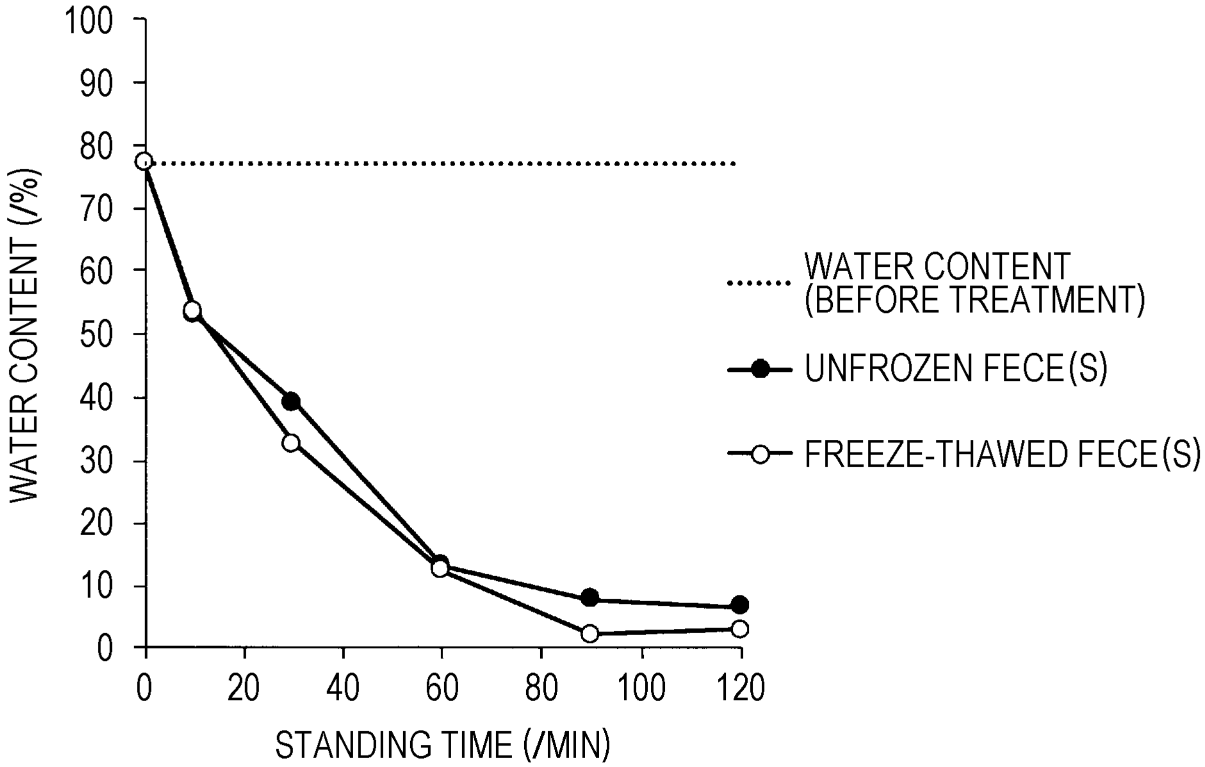
100
90
80
70
60
50
40
30
20
10
0
WATER CONTENT (/%)
0
20
40
60
80
100
120
STANDING TIME (/MIN)
WATER CONTENT (BEFORE TREATMENT)
UNFROZEN FECE(S)
FREEZE-THAWED FECE(S)

FIG. 6

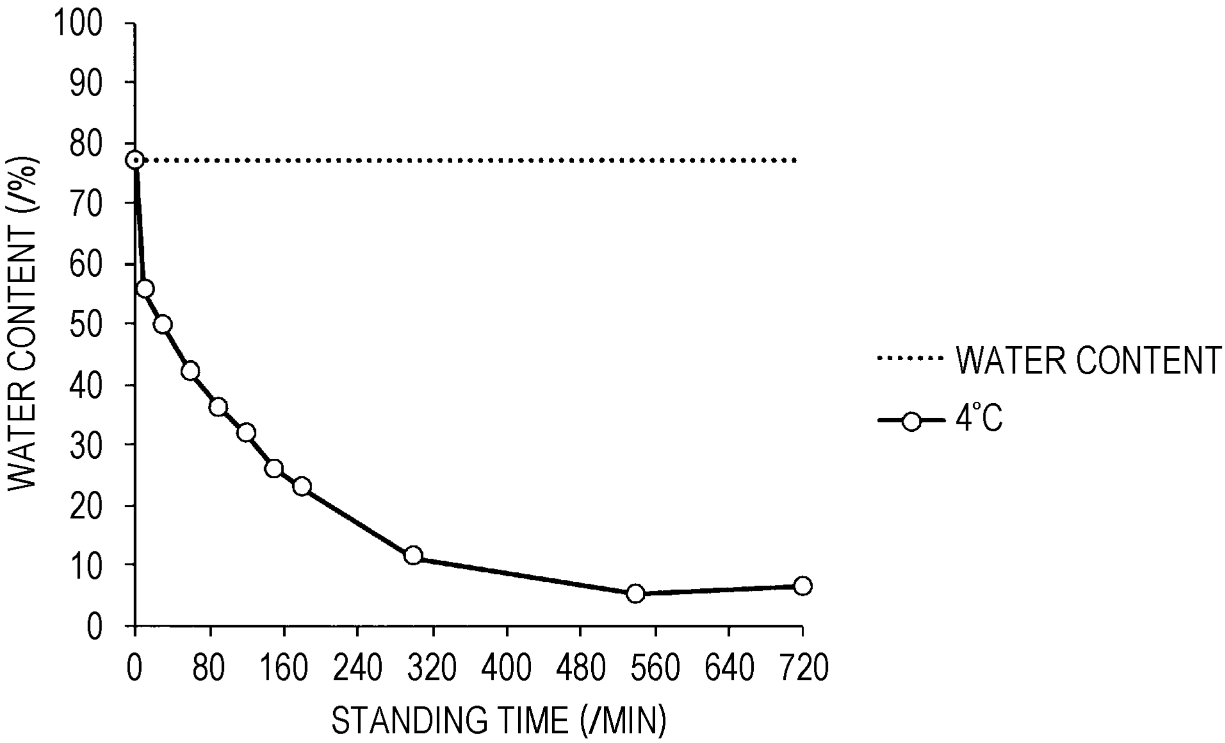
100
90
80
70
60
50
40
30
20
10
0
WATER CONTENT (/%)
0
80
160
240
320
400
480
560
640
720
STANDING TIME (/MIN)
WATER CONTENT
4°C

FIG. 7

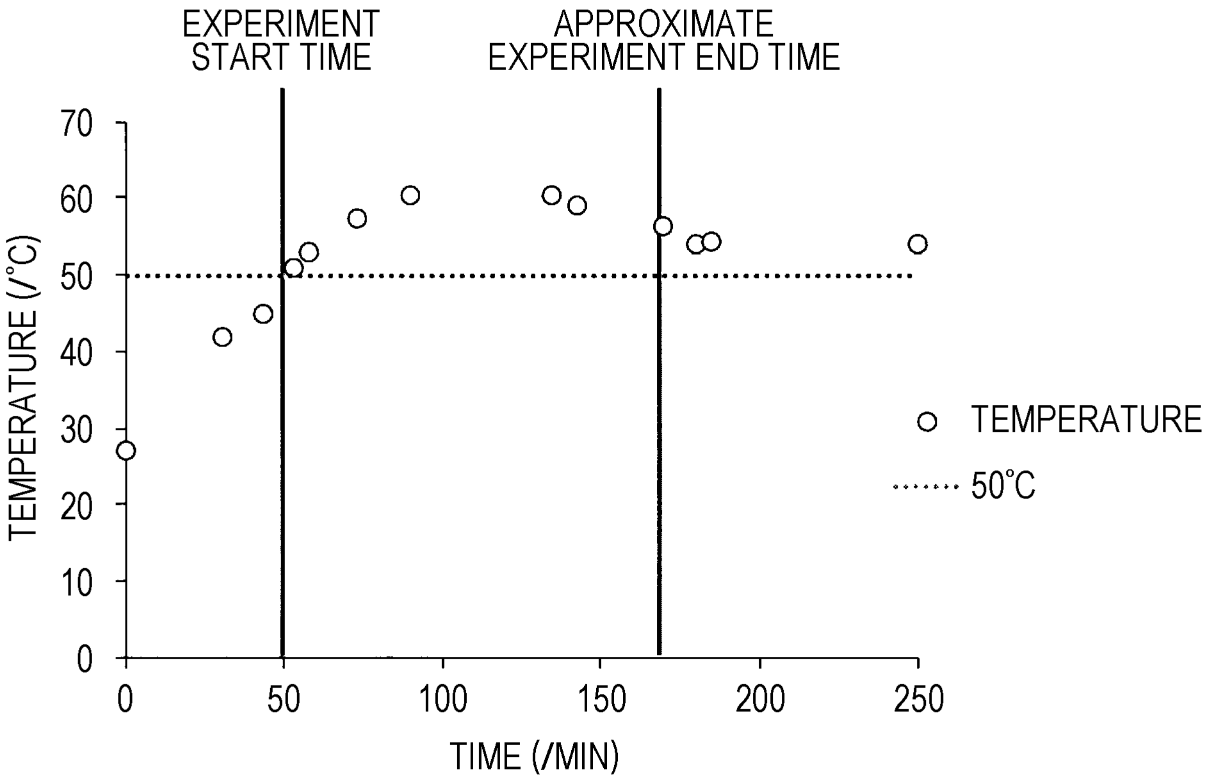
EXPERIMENT
START TIME
APPROXIMATE
EXPERIMENT END TIME
TEMPERATURE (/°C)
70
60
50
40
30
20
10
0
0
50
100
150
200
250
TIME (/MIN)
TEMPERATURE
50°C

FIG. 8

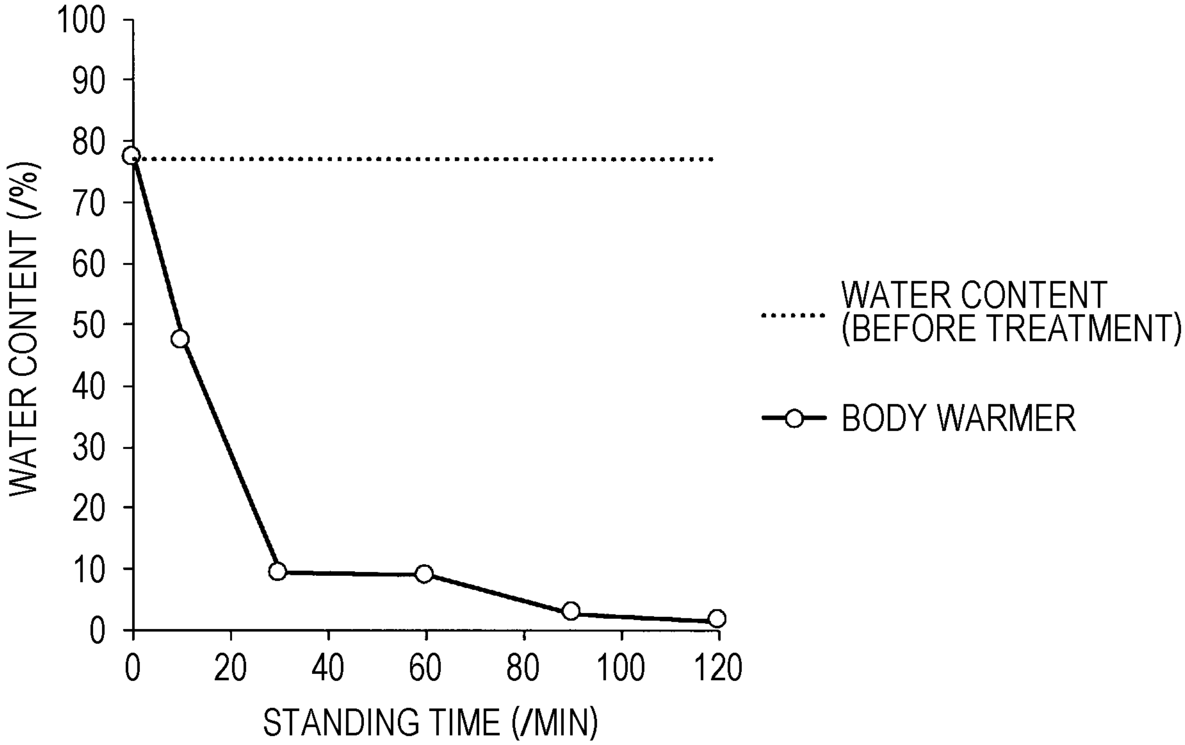
100
90
80
70
60
50
40
30
20
10
0
WATER CONTENT (/%)
0
20
40
60
80
100
120
STANDING TIME (/MIN)
WATER CONTENT
(BEFORE TREATMENT)
BODY WARMER

*FIG. 9*

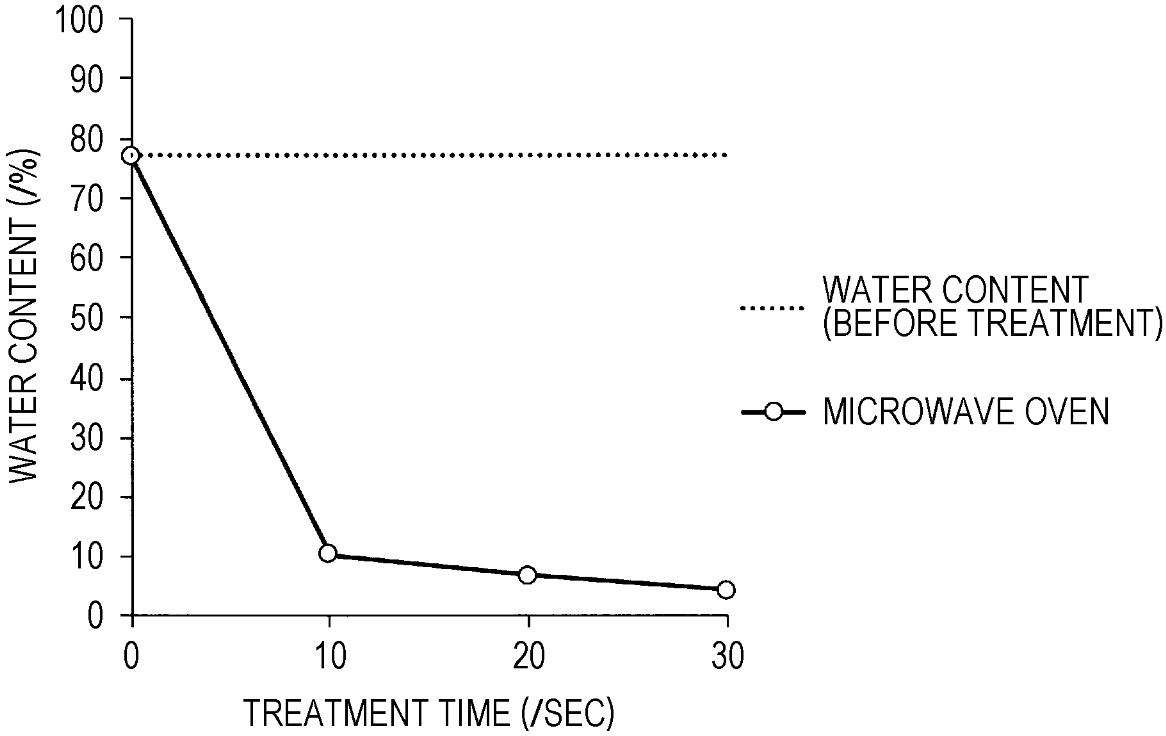
100
90
80
70
60
50
40
30
20
10
0
WATER CONTENT (/%)
0
10
20
30
TREATMENT TIME (/SEC)
WATER CONTENT
(BEFORE TREATMENT)
MICROWAVE OVEN

FIG. 10

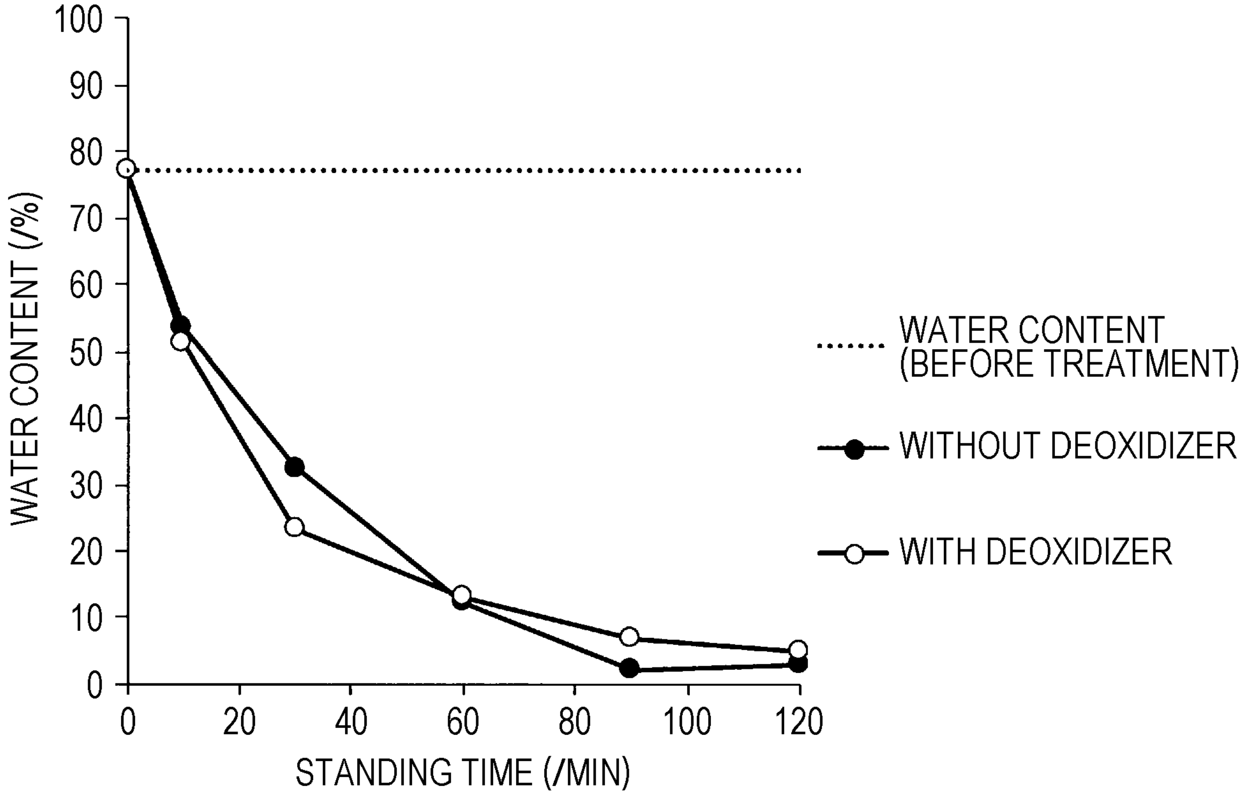
100
90
80
70
60
50
40
30
20
10
0
WATER CONTENT (/%)
0
20
40
60
80
100
120
STANDING TIME (/MIN)
WATER CONTENT (BEFORE TREATMENT)
WITHOUT DEOXIDIZER
WITH DEOXIDIZER

**INTERNATIONAL SEARCH REPORT**

International application No. PCT/JP2019/011001

A. CLASSIFICATION OF SUBJECT MATTER
Int.Cl. C12N1/04(2006.01)i, C12M1/26(2006.01)i, C12Q1/02(2006.01)n

According to International Patent Classification (IPC) or to both national classification and IPC

B. FIELDS SEARCHED

Minimum documentation searched (classification system followed by classification symbols)
Int.Cl. C12N1/04, C12M1/26, C12Q1/02
G01N

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)
CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2017/104132 A1 (KYOWA MEDEX CO., LTD.) 22 June 2017, claims 9, 17-19, paragraph [0011], examples, fig. 2 & US 2019/0011462 A1, claims 9, 17-19, paragraph [0028], examples, fig. 2 & EP 3392639 A1 | 1-5, 7-9, 11-20, 22, 27<br>10<br>6, 21, 23-26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 May 2019 (21.05.2019) | 04 June 2019 (04.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/011001

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | NECHVATAL, J. M. et al., "Fecal collection, ambient preservation,and DNA extraction for PCR amplification of bacterial and human markers from human feces", Journal of Microbiological Methods, 2008, vol. 72, pp. 124-132, in particular, abstract, p. 125, table 2 | 1-5, 7, 8, 11-15, 22, 27<br>9, 10<br>6, 16-21, 23-26 |
| X<br><br>Y<br>A | JP 2011-019505 A (MUKOGAWAGAKUIN EDUCATIONAL INSTITUTION) 03 February 2011, claims 1, 3, paragraph [0070], example 4 (Family: none) | 1-5, 7, 8, 11-15, 22, 27<br>9, 10<br>6, 16-21, 23-26 |
| X<br>A | CN 107586772 A (INSTITUTE OF FOREST ECOLOGY ENVIRONMENT AND PROT CHINESE ACADEMY OF FORESTRY) 16 January 2018, claim 1, paragraphs [0009]-[0013] (Family: none) | 1-5, 11, 22, 27<br>6-10, 12-21, 23-26 |
| Y<br>A | CN 101143757 A (LIN, J. et al.) 19 March 2008, claim 1 (Family: none) | 9, 10<br>1-8, 11-27 |
| A | JP 2008-256389 A (OLYMPUS CORP.) 23 October 2008, entire text (Family: none) | 1-27 |
| A | JP 2001-221721 A (SAPPORO IMUNO DIAGNOSTIC LABORATORY KK) 17 August 2001, entire text (Family: none) | 1-27 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017043087 A


### Non-patent literature cited in the description


- **MURAKAMI et al.** *Evidence-Based Complementary and Alternative Medicine,* 2015, 824395
- **NISHIMOTO et al.** *Gut,* 2016, vol. 65 (9), 1574-5
- **MURAKAMI, S. et al.** The Consumption of Bicarbonate-Rich Mineral Water Improves Glycemic Control. *Evidence-Based Complementary and Alternative Medicine,* 2015, 824395
- **MISHIMA et al.** *Kidney Int.,* September 2017, vol. 92 (3), 634-645